# EUROPEAN PATENT SPECIFICATION

(11) **EP 2 654 781 B1**
(45) Date of publication and mention of the grant of the patent: **24.01.2018**
(21) Application number: 11851933.9
(22) Date of filing: 21.12.2011
(51) Int. Cl.: C07K 16/28, A61P 7/02, A61P 7/06, A61P 37/00

(54) **ANTI-P-SELECTIN ANTIBODIES AND METHODS OF THEIR USE AND IDENTIFICATION**
ANTI-P-SELECTIN-ANTIKÖRPER UND VERFAHREN ZU IHRER VERWENDUNG UND IDENTIFIZIERUNG
ANTICORPS ANTI-P-SÉLECTINE ET LEURS PROCÉDÉS D'UTILISATION ET D'IDENTIFICATION

(30) Priority: 21.12.2010 US 97473910; 31.08.2011 US 201161529682 P
(43) Date of publication of application: 30.10.2013
(60) Divisional application: 17154274.9
(73) Proprietor: Selexys Pharmaceuticals Corporation, Oklahoma City, OK 73104 (US); Oklahoma Medical Research Foundation, Oklahoma City, OK 73104 (US)
(72) Inventor: ROLLINS, Scott, Oklahoma City, OK 73131 (US); ALVAREZ, Richard, Edmond, OK 73013-9007 (US); ROTHER, Russell, Oklahoma City, OK 73151 (US); KAWAR, Ziad, S., Oklahoma City, OK 73120 (US); MCEVER, Rodger, P., Oklahoma City, OK 73120 (US)
(74) Representative: HGF Limited
(86) International application number: PCT/US2011/066470
(87) International publication number: WO 2012/088265

(56) References cited:
- WO-A2-2008/069999
- US-A- 5 593 882
- US-A1- 2009 285 812
- US-A1- 2010 209 423
- US-A1- 2011 212 096
- US-A1- 2011 287 017
- US-A1- 2011 293 617
- MOORE ET AL.: 'P-selectin glycoprotein ligand-1 mediates rolling of human neutrophils on P- selectin.' J CELL BIOL vol. 128, no. 4, February 1995, pages 661 - 671, XP007916694

### SEQUENCE LISTING

<110> Selexys Pharmaceuticals Corporation Oklahoma Medical Research Foundation Rollins, Scott Alvarez, Richard Rother, Russell Kawar, Ziad S. McEver, Rodger P.
<120> ANTI-P-SELECTIN ANTIBODIES AND METHODS OF THEIR USE AND IDENTIFICATION
<130> 7149.015wo
<150> 61/529,682
   <151> 2011-08-31
<150> 12/974,739
   <151> 2010-12-21
<160> 10
<170> PatentIn version 3.5
<210> 1
   <211> 279
   <212> PRT
   <213> Homo sapiens
<400> 1
   Trp Thr Tyr His Tyr Ser Thr Lys Ala Tyr Ser Trp Asn Ile Ser Arg
   1               5                   10                  15
   Lys Tyr Cys Gln Asn Arg Tyr Thr Asp Leu Val Ala Ile Gln Asn Lys
               20                  25                  30
   Asn Glu Ile Asp Tyr Leu Asn Lys Val Leu Pro Tyr Tyr Ser Ser Tyr
           35                  40                  45
   Tyr Trp Ile Gly Ile Arg Lys Asn Asn Lys Thr Trp Thr Trp Val Gly
       50                  55                  60
   Thr Lys Lys Ala Leu Thr Asn Glu Ala Glu Asn Trp Ala Asp Asn Glu
   65                  70                  75                  80
   Pro Asn Asn Lys Arg Asn Asn Glu Asp Cys Val Glu Ile Tyr Ile Lys
                   85                  90                  95
   Ser Pro Ser Ala Pro Gly Lys Trp Asn Asp Glu His Cys Leu Lys Lys
               100                 105                 110
   Lys His Ala Leu Cys Tyr Thr Ala Ser Cys Gln Asp Met Ser Cys Ser
           115                 120                 125
   Lys Gln Gly Glu Cys Leu Glu Thr Ile Gly Asn Tyr Thr Cys Ser Cys
       130                 135                 140
   Tyr Pro Gly Phe Tyr Gly Pro Glu Cys Glu Tyr Val Arg Glu Cys Gly
   145                 150                 155                 160
   Glu Leu Glu Leu Pro Gln His Val Leu Met Asn Cys Ser His Pro Leu
                   165                 170                 175
   Gly Asn Phe Ser Phe Asn Ser Gln Cys Ser Phe His Cys Thr Asp Gly
               180                 185                 190
   Tyr Gln Val Asn Gly Pro Ser Lys Leu Glu Cys Leu Ala Ser Gly Ile
           195                 200                 205
   Trp Thr Asn Lys Pro Pro Gln Cys Leu Ala Ala Gln Cys Pro Pro Leu
       210                 215                 220
   Lys Ile Pro Glu Arg Gly Asn Met Thr Cys Leu His Ser Ala Lys Ala
   225                 230                 235                 240
   Phe Gln His Gln Ser Ser Cys Ser Phe Ser Cys Glu Glu Gly Phe Ala
                   245                 250                 255
   Leu Val Gly Pro Glu Val Val Gln Cys Thr Ala Ser Gly Val Trp Thr
               260                 265                 270
   Ala Pro Ala Pro Val Cys Lys
           275
<210> 2
   <211> 282
   <212> PRT
   <213> Mus musculus
<400> 2
   Trp Thr Tyr Asn Tyr Ser Thr Lys Ala Tyr Ser Trp Asn Asn Ser Arg
   1               5                   10                  15
   Val Phe Cys Arg Arg His Phe Thr Asp Leu Val Ala Ile Gln Asn Lys
               20                  25                  30
   Asn Glu Ile Ala His Leu Asn Asp Val Ile Pro Phe Phe Asn Ser Tyr
           35                  40                  45
   Tyr Trp Ile Gly Ile Arg Lys Ile Asn Asn Lys Trp Thr Trp Val Gly
       50                  55                  60
   Thr Asn Lys Thr Leu Thr Glu Glu Ala Glu Asn Trp Ala Asp Asn Glu
   65                  70                  75                  80
   Pro Asn Asn Lys Lys Asn Asn Gln Asp Cys Val Glu Ile Tyr Ile Lys
                   85                  90                  95
   Ser Asn Ser Ala Pro Gly Lys Trp Asn Asp Glu Pro Cys Phe Lys Arg
               100                 105                 110
   Lys Arg Ala Leu Cys Tyr Thr Ala Ser Cys Gln Asp Met Ser Cys Ser
           115                 120                 125
   Asn Gln Gly Glu Cys Ile Glu Thr Ile Gly Ser Tyr Thr Cys Ser Cys
       130                 135                 140
   Tyr Pro Gly Phe Tyr Gly Pro Glu Cys Glu Tyr Val Lys Glu Cys Gly
   145                 150                 155                 160
   Lys Val Asn Ile Pro Gln His Val Leu Met Asn Cys Ser His Pro Leu
                   165                 170                 175
   Gly Glu Phe Ser Phe Asn Ser Gln Cys Thr Phe Ser Cys Ala Glu Gly
               180                 185                 190
   Tyr Glu Leu Asp Gly Pro Gly Glu Leu Gln Cys Leu Ala Ser Gly Ile
           195                 200                 205
   Trp Thr Asn Asn Pro Pro Lys Cys Asp Ala Val Gln Cys Gln Ser Leu
       210                 215                 220
   Glu Ala Pro Pro His Gly Thr Met Ala Cys Met His Pro Ile Ala Ala
   225                 230                 235                 240
   Phe Ala Tyr Asp Ser Ser Cys Lys Phe Glu Cys Gln Pro Gly Tyr Arg
                   245                 250                 255
   Ala Arg Gly Ser Asn Thr Leu His Cys Thr Gly Ser Gly Gln Trp Ser
               260                 265                 270
   Glu Pro Leu Pro Thr Cys Glu Ala Ile Ala
           275                 280
<210> 3
   <211> 279
   <212> PRT
   <213> Artificial Sequence
<220>
   <223> Chimera-1 (mouse P-selecin amino acids substituted into human P-selectin at positions 4, 14, 17, 18, 20, 21, 22, 23)
<220>
   <221> MISC_FEATURE
   <222> (4)..(4)
   <223> H to N
<220>
   <221> MISC_FEATURE
   <222> (14)..(14)
   <223> I to N
<220>
   <221> MISC_FEATURE
   <222> (17)..(17)
   <223> K to V
<220>
   <221> MISC_FEATURE
   <222> (18)..(18)
   <223> Y to F
<220>
   <221> MISC_FEATURE
   <222> (20)..(20)
   <223> Q to R
<220>
   <221> MISC_FEATURE
   <222> (21)..(21)
   <223> N to R
<220>
   <221> MISC_FEATURE
   <222> (22)..(22)
   <223> R to H
<220>
   <221> MISC_FEATURE
   <222> (23)..(23)
   <223> Y to F
<400> 3
   Trp Thr Tyr Asn Tyr Ser Thr Lys Ala Tyr Ser Trp Asn Asn Ser Arg
   1               5                   10                  15
   Val Phe Cys Arg Arg His Phe Thr Asp Leu Val Ala Ile Gln Asn Lys
               20                  25                  30
   Asn Glu Ile Asp Tyr Leu Asn Lys Val Leu Pro Tyr Tyr Ser Ser Tyr
           35                  40                  45
   Tyr Trp Ile Gly Ile Arg Lys Asn Asn Lys Thr Trp Thr Trp Val Gly
       50                  55                  60
   Thr Lys Lys Ala Leu Thr Asn Glu Ala Glu Asn Trp Ala Asp Asn Glu
   65                  70                  75                  80
   Pro Asn Asn Lys Arg Asn Asn Glu Asp Cys Val Glu Ile Tyr Ile Lys
                   85                  90                  95
   Ser Pro Ser Ala Pro Gly Lys Trp Asn Asp Glu His Cys Leu Lys Lys
               100                 105                 110
   Lys His Ala Leu Cys Tyr Thr Ala Ser Cys Gln Asp Met Ser Cys Ser
           115                 120                 125
   Lys Gln Gly Glu Cys Leu Glu Thr Ile Gly Asn Tyr Thr Cys Ser Cys
       130                 135                 140
   Tyr Pro Gly Phe Tyr Gly Pro Glu Cys Glu Tyr Val Arg Glu Cys Gly
   145                 150                 155                 160
   Glu Leu Glu Leu Pro Gln His Val Leu Met Asn Cys Ser His Pro Leu
                   165                 170                 175
   Gly Asn Phe Ser Phe Asn Ser Gln Cys Ser Phe His Cys Thr Asp Gly
               180                 185                 190
   Tyr Gln Val Asn Gly Pro Ser Lys Leu Glu Cys Leu Ala Ser Gly Ile
           195                 200                 205
   Trp Thr Asn Lys Pro Pro Gln Cys Leu Ala Ala Gln Cys Pro Pro Leu
       210                 215                 220
   Lys Ile Pro Glu Arg Gly Asn Met Thr Cys Leu His Ser Ala Lys Ala
   225                 230                 235                 240
   Phe Gln His Gln Ser Ser Cys Ser Phe Ser Cys Glu Glu Gly Phe Ala
                   245                 250                 255
   Leu Val Gly Pro Glu Val Val Gln Cys Thr Ala Ser Gly Val Trp Thr
               260                 265                 270
   Ala Pro Ala Pro Val Cys Lys
           275
<210> 4
   <211> 282
   <212> PRT
   <213> Artificial Sequence
<220>
   <223> Chimera-2 (human P-selectin positions 1-35; mouse P-selectin thereafter)
<400> 4
   Trp Thr Tyr His Tyr Ser Thr Lys Ala Tyr Ser Trp Asn Ile Ser Arg
   1               5                   10                  15
   Lys Tyr Cys Gln Asn Arg Tyr Thr Asp Leu Val Ala Ile Gln Asn Lys
               20                  25                  30
   Asn Glu Ile Ala His Leu Asn Asp Val Ile Pro Phe Phe Asn Ser Tyr
           35                  40                  45
   Tyr Trp Ile Gly Ile Arg Lys Ile Asn Asn Lys Trp Thr Trp Val Gly
       50                  55                  60
   Thr Asn Lys Thr Leu Thr Glu Glu Ala Glu Asn Trp Ala Asp Asn Glu
   65                  70                  75                  80
   Pro Asn Asn Lys Lys Asn Asn Gln Asp Cys Val Glu Ile Tyr Ile Lys
                   85                  90                  95
   Ser Asn Ser Ala Pro Gly Lys Trp Asn Asp Glu Pro Cys Phe Lys Arg
               100                 105                 110
   Lys Arg Ala Leu Cys Tyr Thr Ala Ser Cys Gln Asp Met Ser Cys Ser
           115                 120                 125
   Asn Gln Gly Glu Cys Ile Glu Thr Ile Gly Ser Tyr Thr Cys Ser Cys
       130                 135                 140
   Tyr Pro Gly Phe Tyr Gly Pro Glu Cys Glu Tyr Val Lys Glu Cys Gly
   145                 150                 155                 160
   Lys Val Asn Ile Pro Gln His Val Leu Met Asn Cys Ser His Pro Leu
                   165                 170                 175
   Gly Glu Phe Ser Phe Asn Ser Gln Cys Thr Phe Ser Cys Ala Glu Gly
               180                 185                 190
   Tyr Glu Leu Asp Gly Pro Gly Glu Leu Gln Cys Leu Ala Ser Gly Ile
           195                 200                 205
   Trp Thr Asn Asn Pro Pro Lys Cys Asp Ala Val Gln Cys Gln Ser Leu
       210                 215                 220
   Glu Ala Pro Pro His Gly Thr Met Ala Cys Met His Pro Ile Ala Ala
   225                 230                 235                 240
   Phe Ala Tyr Asp Ser Ser Cys Lys Phe Glu Cys Gln Pro Gly Tyr Arg
                   245                 250                 255
   Ala Arg Gly Ser Asn Thr Leu His Cys Thr Gly Ser Gly Gln Trp Ser
               260                 265                 270
   Glu Pro Leu Pro Thr Cys Glu Ala Ile Ala
           275                 280
<210> 5
   <211> 279
   <212> PRT
   <213> Artificial Sequence
<220>
   <223> Chimera-3 (single amino acid change in human P-selectin - human H4 to mouse N4)
<220>
   <221> MISC_FEATURE
   <222> (4)..(4)
   <223> H to N
<400> 5
   Trp Thr Tyr Asn Tyr Ser Thr Lys Ala Tyr Ser Trp Asn Ile Ser Arg
   1               5                   10                  15
   Lys Tyr Cys Gln Asn Arg Tyr Thr Asp Leu Val Ala Ile Gln Asn Lys
               20                  25                  30
   Asn Glu Ile Asp Tyr Leu Asn Lys Val Leu Pro Tyr Tyr Ser Ser Tyr
           35                  40                  45
   Tyr Trp Ile Gly Ile Arg Lys Asn Asn Lys Thr Trp Thr Trp Val Gly
       50                  55                  60
   Thr Lys Lys Ala Leu Thr Asn Glu Ala Glu Asn Trp Ala Asp Asn Glu
   65                  70                  75                  80
   Pro Asn Asn Lys Arg Asn Asn Glu Asp Cys Val Glu Ile Tyr Ile Lys
                   85                  90                  95
   Ser Pro Ser Ala Pro Gly Lys Trp Asn Asp Glu His Cys Leu Lys Lys
               100                 105                 110
   Lys His Ala Leu Cys Tyr Thr Ala Ser Cys Gln Asp Met Ser Cys Ser
           115                 120                 125
   Lys Gln Gly Glu Cys Leu Glu Thr Ile Gly Asn Tyr Thr Cys Ser Cys
       130                 135                 140
   Tyr Pro Gly Phe Tyr Gly Pro Glu Cys Glu Tyr Val Arg Glu Cys Gly
   145                 150                 155                 160
   Glu Leu Glu Leu Pro Gln His Val Leu Met Asn Cys Ser His Pro Leu
                   165                 170                 175
   Gly Asn Phe Ser Phe Asn Ser Gln Cys Ser Phe His Cys Thr Asp Gly
               180                 185                 190
   Tyr Gln Val Asn Gly Pro Ser Lys Leu Glu Cys Leu Ala Ser Gly Ile
           195                 200                 205
   Trp Thr Asn Lys Pro Pro Gln Cys Leu Ala Ala Gln Cys Pro Pro Leu
       210                 215                 220
   Lys Ile Pro Glu Arg Gly Asn Met Thr Cys Leu His Ser Ala Lys Ala
   225                 230                 235                 240
   Phe Gln His Gln Ser Ser Cys Ser Phe Ser Cys Glu Glu Gly Phe Ala
                   245                 250                 255
   Leu Val Gly Pro Glu Val Val Gln Cys Thr Ala Ser Gly Val Trp Thr
               260                 265                 270
   Ala Pro Ala Pro Val Cys Lys
           275
<210> 6
   <211> 279
   <212> PRT
   <213> Artificial Sequence
<220>
   <223> Chimera-4Q (substutituion of Q for H4 in human P-selectin removes putative glycosylation site)
<220>
   <221> MISC_FEATURE
   <222> (4)..(4)
   <223> H to Q
<400> 6
   Trp Thr Tyr Gln Tyr Ser Thr Lys Ala Tyr Ser Trp Asn Ile Ser Arg
   1               5                   10                  15
   Lys Tyr Cys Gln Asn Arg Tyr Thr Asp Leu Val Ala Ile Gln Asn Lys
               20                  25                  30
   Asn Glu Ile Asp Tyr Leu Asn Lys Val Leu Pro Tyr Tyr Ser Ser Tyr
           35                  40                  45
   Tyr Trp Ile Gly Ile Arg Lys Asn Asn Lys Thr Trp Thr Trp Val Gly
       50                  55                  60
   Thr Lys Lys Ala Leu Thr Asn Glu Ala Glu Asn Trp Ala Asp Asn Glu
   65                  70                  75                  80
   Pro Asn Asn Lys Arg Asn Asn Glu Asp Cys Val Glu Ile Tyr Ile Lys
                   85                  90                  95
   Ser Pro Ser Ala Pro Gly Lys Trp Asn Asp Glu His Cys Leu Lys Lys
               100                 105                 110
   Lys His Ala Leu Cys Tyr Thr Ala Ser Cys Gln Asp Met Ser Cys Ser
           115                 120                 125
   Lys Gln Gly Glu Cys Leu Glu Thr Ile Gly Asn Tyr Thr Cys Ser Cys
       130                 135                 140
   Tyr Pro Gly Phe Tyr Gly Pro Glu Cys Glu Tyr Val Arg Glu Cys Gly
   145                 150                 155                 160
   Glu Leu Glu Leu Pro Gln His Val Leu Met Asn Cys Ser His Pro Leu
                   165                 170                 175
   Gly Asn Phe Ser Phe Asn Ser Gln Cys Ser Phe His Cys Thr Asp Gly
               180                 185                 190
   Tyr Gln Val Asn Gly Pro Ser Lys Leu Glu Cys Leu Ala Ser Gly Ile
           195                 200                 205
   Trp Thr Asn Lys Pro Pro Gln Cys Leu Ala Ala Gln Cys Pro Pro Leu
       210                 215                 220
   Lys Ile Pro Glu Arg Gly Asn Met Thr Cys Leu His Ser Ala Lys Ala
   225                 230                 235                 240
   Phe Gln His Gln Ser Ser Cys Ser Phe Ser Cys Glu Glu Gly Phe Ala
                   245                 250                 255
   Leu Val Gly Pro Glu Val Val Gln Cys Thr Ala Ser Gly Val Trp Thr
               260                 265                 270
   Ala Pro Ala Pro Val Cys Lys
           275
<210> 7
   <211> 282
   <212> PRT
   <213> Artificial Sequence
<220>
   <223> Chimera-5 (human P-selectin positions 1-121; thereafter mouse P-selectin)
<400> 7
   Trp Thr Tyr His Tyr Ser Thr Lys Ala Tyr Ser Trp Asn Ile Ser Arg
   1               5                   10                  15
   Lys Tyr Cys Gln Asn Arg Tyr Thr Asp Leu Val Ala Ile Gln Asn Lys
               20                  25                  30
   Asn Glu Ile Asp Tyr Leu Asn Lys Val Leu Pro Tyr Tyr Ser Ser Tyr
           35                  40                  45
   Tyr Trp Ile Gly Ile Arg Lys Asn Asn Lys Thr Trp Thr Trp Val Gly
       50                  55                  60
   Thr Lys Lys Ala Leu Thr Asn Glu Ala Glu Asn Trp Ala Asp Asn Glu
   65                  70                  75                  80
   Pro Asn Asn Lys Arg Asn Asn Glu Asp Cys Val Glu Ile Tyr Ile Lys
                   85                  90                  95
   Ser Pro Ser Ala Pro Gly Lys Trp Asn Asp Glu His Cys Leu Lys Lys
               100                 105                 110
   Lys His Ala Leu Cys Tyr Thr Ala Ser Cys Gln Asp Met Ser Cys Ser
           115                 120                 125
   Asn Gln Gly Glu Cys Ile Glu Thr Ile Gly Ser Tyr Thr Cys Ser Cys
       130                 135                 140
   Tyr Pro Gly Phe Tyr Gly Pro Glu Cys Glu Tyr Val Lys Glu Cys Gly
   145                 150                 155                 160
   Lys Val Asn Ile Pro Gln His Val Leu Met Asn Cys Ser His Pro Leu
                   165                 170                 175
   Gly Glu Phe Ser Phe Asn Ser Gln Cys Thr Phe Ser Cys Ala Glu Gly
               180                 185                 190
   Tyr Glu Leu Asp Gly Pro Gly Glu Leu Gln Cys Leu Ala Ser Gly Ile
           195                 200                 205
   Trp Thr Asn Asn Pro Pro Lys Cys Asp Ala Val Gln Cys Gln Ser Leu
       210                 215                 220
   Glu Ala Pro Pro His Gly Thr Met Ala Cys Met His Pro Ile Ala Ala
   225                 230                 235                 240
   Phe Ala Tyr Asp Ser Ser Cys Lys Phe Glu Cys Gln Pro Gly Tyr Arg
                   245                 250                 255
   Ala Arg Gly Ser Asn Thr Leu His Cys Thr Gly Ser Gly Gln Trp Ser
               260                 265                 270
   Glu Pro Leu Pro Thr Cys Glu Ala Ile Ala
           275                 280
<210> 8
   <211> 282
   <212> PRT
   <213> Artificial Sequence
<220>
   <223> Chimera-6 (human P-selectin positions 1-177; thereafter mouse P-selectin)
<400> 8
   Trp Thr Tyr His Tyr Ser Thr Lys Ala Tyr Ser Trp Asn Ile Ser Arg
   1               5                   10                  15
   Lys Tyr Cys Gln Asn Arg Tyr Thr Asp Leu Val Ala Ile Gln Asn Lys
               20                  25                  30
   Asn Glu Ile Asp Tyr Leu Asn Lys Val Leu Pro Tyr Tyr Ser Ser Tyr
           35                  40                  45
   Tyr Trp Ile Gly Ile Arg Lys Asn Asn Lys Thr Trp Thr Trp Val Gly
       50                  55                  60
   Thr Lys Lys Ala Leu Thr Asn Glu Ala Glu Asn Trp Ala Asp Asn Glu
   65                  70                  75                  80
   Pro Asn Asn Lys Arg Asn Asn Glu Asp Cys Val Glu Ile Tyr Ile Lys
                   85                  90                  95
   Ser Pro Ser Ala Pro Gly Lys Trp Asn Asp Glu His Cys Leu Lys Lys
               100                 105                 110
   Lys His Ala Leu Cys Tyr Thr Ala Ser Cys Gln Asp Met Ser Cys Ser
           115                 120                 125
   Lys Gln Gly Glu Cys Leu Glu Thr Ile Gly Asn Tyr Thr Cys Ser Cys
       130                 135                 140
   Tyr Pro Gly Phe Tyr Gly Pro Glu Cys Glu Tyr Val Arg Glu Cys Gly
   145                 150                 155                 160
   Glu Leu Glu Leu Pro Gln His Val Leu Met Asn Cys Ser His Pro Leu
                   165                 170                 175
   Gly Glu Phe Ser Phe Asn Ser Gln Cys Thr Phe Ser Cys Ala Glu Gly
               180                 185                 190
   Tyr Glu Leu Asp Gly Pro Gly Glu Leu Gln Cys Leu Ala Ser Gly Ile
           195                 200                 205
   Trp Thr Asn Asn Pro Pro Lys Cys Asp Ala Val Gln Cys Gln Ser Leu
       210                 215                 220
   Glu Ala Pro Pro His Gly Thr Met Ala Cys Met His Pro Ile Ala Ala
   225                 230                 235                 240
   Phe Ala Tyr Asp Ser Ser Cys Lys Phe Glu Cys Gln Pro Gly Tyr Arg
                   245                 250                 255
   Ala Arg Gly Ser Asn Thr Leu His Cys Thr Gly Ser Gly Gln Trp Ser
               260                 265                 270
   Glu Pro Leu Pro Thr Cys Glu Ala Ile Ala
           275                 280
<210> 9
   <211> 282
   <212> PRT
   <213> Artificial Sequence
<220>
   <223> Chimera-7 (human P-selectin positions 1-97; thereafter mouse P-selectin)
<400> 9
   Trp Thr Tyr His Tyr Ser Thr Lys Ala Tyr Ser Trp Asn Ile Ser Arg
   1               5                   10                  15
   Lys Tyr Cys Gln Asn Arg Tyr Thr Asp Leu Val Ala Ile Gln Asn Lys
               20                  25                  30
   Asn Glu Ile Asp Tyr Leu Asn Lys Val Leu Pro Tyr Tyr Ser Ser Tyr
           35                  40                  45
   Tyr Trp Ile Gly Ile Arg Lys Asn Asn Lys Thr Trp Thr Trp Val Gly
   50 55 60
   Thr Lys Lys Ala Leu Thr Asn Glu Ala Glu Asn Trp Ala Asp Asn Glu
   65 70 75 80
   Pro Asn Asn Lys Arg Asn Asn Glu Asp Cys Val Glu Ile Tyr Ile Lys
   85 90 95
   Ser Asn Ser Ala Pro Gly Lys Trp Asn Asp Glu Pro Cys Phe Lys Arg
   100 105 110
   Lys Arg Ala Leu Cys Tyr Thr Ala Ser Cys Gln Asp Met Ser Cys Ser
   115 120 125
   Asn Gln Gly Glu Cys Ile Glu Thr Ile Gly Ser Tyr Thr Cys Ser Cys
   130 135 140
   Tyr Pro Gly Phe Tyr Gly Pro Glu Cys Glu Tyr Val Lys Glu Cys Gly
   145 150 155 160
   Lys Val Asn Ile Pro Gln His Val Leu Met Asn Cys Ser His Pro Leu
   165 170 175
   Gly Glu Phe Ser Phe Asn Ser Gln Cys Thr Phe Ser Cys Ala Glu Gly
   180 185 190
   Tyr Glu Leu Asp Gly Pro Gly Glu Leu Gln Cys Leu Ala Ser Gly Ile
   195 200 205
   Trp Thr Asn Asn Pro Pro Lys Cys Asp Ala Val Gln Cys Gln Ser Leu
   210 215 220
   Glu Ala Pro Pro His Gly Thr Met Ala Cys Met His Pro Ile Ala Ala
   225 230 235 240
   Phe Ala Tyr Asp Ser Ser Cys Lys Phe Glu Cys Gln Pro Gly Tyr Arg
   245 250 255
   Ala Arg Gly Ser Asn Thr Leu His Cys Thr Gly Ser Gly Gln Trp Ser
   260 265 270
   Glu Pro Leu Pro Thr Cys Glu Ala Ile Ala
   275 280
<210> 10
   <211> 282
   <212> PRT
   <213> Artificial Sequence
<220>
   <223> Chimera-8 (human P-selectin amino acids substituted into mouse P-selectin at positions 4, 14, 17, 21, 22)
<220>
   <221> MISC_FEATURE
   <222> (4)..(4)
   <223> N to H
<220>
   <221> MISC_FEATURE
   <222> (14)..(14)
   <223> N to I
<220>
   <221> MISC_FEATURE
   <222> (17)..(17)
   <223> V to K
<220>
   <221> MISC_FEATURE
   <222> (21)..(21)
   <223> R to N
<220>
   <221> MISC_FEATURE
   <222> (22)..(22)
   <223> H to R
<400> 10
   Trp Thr Tyr His Tyr Ser Thr Lys Ala Tyr Ser Trp Asn Ile Ser Arg
   1               5                   10                  15
   Lys Phe Cys Arg Asn Arg Phe Thr Asp Leu Val Ala Ile Gln Asn Lys
               20                  25                  30
   Asn Glu Ile Ala His Leu Asn Asp Val Ile Pro Phe Phe Asn Ser Tyr
           35                  40                  45
   Tyr Trp Ile Gly Ile Arg Lys Ile Asn Asn Lys Trp Thr Trp Val Gly
       50                  55                  60
   Thr Asn Lys Thr Leu Thr Glu Glu Ala Glu Asn Trp Ala Asp Asn Glu
   65                  70                  75                  80
   Pro Asn Asn Lys Lys Asn Asn Gln Asp Cys Val Glu Ile Tyr Ile Lys
                   85                  90                  95
   Ser Asn Ser Ala Pro Gly Lys Trp Asn Asp Glu Pro Cys Phe Lys Arg
               100                 105                 110
   Lys Arg Ala Leu Cys Tyr Thr Ala Ser Cys Gln Asp Met Ser Cys Ser
           115                 120                 125
   Asn Gln Gly Glu Cys Ile Glu Thr Ile Gly Ser Tyr Thr Cys Ser Cys
       130                 135                 140
   Tyr Pro Gly Phe Tyr Gly Pro Glu Cys Glu Tyr Val Lys Glu Cys Gly
   145                 150                 155                 160
   Lys Val Asn Ile Pro Gln His Val Leu Met Asn Cys Ser His Pro Leu
                   165                 170                 175
   Gly Glu Phe Ser Phe Asn Ser Gln Cys Thr Phe Ser Cys Ala Glu Gly
               180                 185                 190
   Tyr Glu Leu Asp Gly Pro Gly Glu Leu Gln Cys Leu Ala Ser Gly Ile
           195                 200                 205
   Trp Thr Asn Asn Pro Pro Lys Cys Asp Ala Val Gln Cys Gln Ser Leu
       210                 215                 220
   Glu Ala Pro Pro His Gly Thr Met Ala Cys Met His Pro Ile Ala Ala
   225                 230                 235                 240
   Phe Ala Tyr Asp Ser Ser Cys Lys Phe Glu Cys Gln Pro Gly Tyr Arg
                   245                 250                 255
   Ala Arg Gly Ser Asn Thr Leu His Cys Thr Gly Ser Gly Gln Trp Ser
               260                 265                 270
   Glu Pro Leu Pro Thr Cys Glu Ala Ile Ala
           275                 280

## Description

The present invention relates to antibodies and antibody fragments which bind to specific conformational epitopes of P-selectin, and to methods of their use and identification.

In normal hemostasis and immune surveillance, leukocytes circulate freely in the blood and respond to injury and infection in a sequential process of adhesion signaling mediated by cell adhesion molecules (1-3). In inflammatory and thrombotic disease, this process is dysregulated and can sustain pathology wherein leukocytes attack the body's own tissue and can cause serious and sometimes deadly complications. It is well known that leukocyte adhesion plays a major role in the pathology of many inflammatory and thrombotic disorders such as vasoocclusion in sickle cell disease, reperfusion injury, thrombosis, atherosclerosis, asthma, rheumatoid arthritis, psoriasis and tumor metastasis (4-15) deep venous thrombosis (DVT). P-selectin is also involved in other disease processes, such as tissue and organ damage associated with inflammation, e.g., ischemia-reperfusion injury. P-selectin is thus a target for intervention in human inflammatory and thrombotic diseases.

Selectins are a family of adhesion proteins which are known to play key roles in the recruitment of leukocytes to activated endothelium and platelets. P-selectin is a member of the selectin family of adhesion glycoproteins which also includes L- and E-selectins (16). The selectins mediate the recruitment, initial tethering and rolling, and adherence of leukocytes to sites of inflammation (1). P-selectin is stored in Weibel-Palade bodies of endothelial cells and alpha-granules of platelets and is rapidly mobilized to the plasma membrane upon stimulation by vasoactive substances such as histamine and thrombin (17).

### Sickle Cell Disease

Sickle cell disease (SCD) is a rare inherited blood disorder that causes chronic anemia and vasoocclusion, affecting primarily people of African-American heritage in the United States. Sickle cell disease is the most common single gene disorder in African Americans, affecting approximately 1 in 375-600 persons of African ancestry (18, 19). Sickle cell conditions are also common among people of Mediterranean countries, Africa the Caribbean and parts of South and Central America (18, 19).

SCD is an autosomal recessive disease caused by a single missense mutation (Val6Ala) in the β-globin gene that renders the mutant hemoglobin less soluble and prone to polymerization upon deoxygenation. The polymerization of hemoglobin causes deformation of the erythrocyte to give the cell a sickled shape (20).

SCD has three common variants: homozygous sickle cell disease (hemoglobin SS disease), doubly heterozygous sickle hemoglobin C disease (hemoglobin SC disease) and the sickle β-thalassemias. The most common and severe form of the disease occurs in individuals who inherit two copies of the HbS variant (HbSS) and the primary hemoglobin in their red blood cells is sickle hemoglobin. Other individuals can be affected as compound heterozygotes with varying severity of the disease. They have one copy of the HbS variant paired with a copy of another β-globin gene variant. HBSC results in a mild form of the disease. Hb β-thalassemia variants (resulting in the inability to produce the normal βA globin chain (β°) or a reduction in its production (β⁺) result in a range of clinical severities. HbS β⁰ is a severe form, whereas HbS β⁺ can be moderate or mild based on the contribution of each variant to the total hemoglobin of the patient. Other more rare variants can result if in conjunction with the S gene, another abnormal hemoglobin is inherited from the other parent, such as D, G or O. The predominant form of sickle cell is present in individuals with one copy of HbS and one copy of the normal β-globin gene (HbA). These individuals carry the sickle cell trait (18).

SCD affects an estimated 50,000-100,000 people in the US (21-24). All individuals that are homozygous or compound heterozygous for HbS show some clinical manifestations of SCD. Symptoms usually appear within the first 6 months of life but there is considerable variability in SCD severity (25). Individuals with HbSS are most severely affected, followed by individuals with HBbS β°-thalassemia (22, 26). In addition to genotype, additional factors affect disease severity such as the levels of fetal hemoglobin and the haplotype of the β-globin cluster, a region that contains 5 genes that code for the β portion of hemoglobin. Despite the capacity to determine genetic risk factors, the ability to predict disease course from birth is limited (27).

In the USA, sickle cell screening at birth is mandated in all 50 states and the District of Columbia (28) and offers an opportunity for early intervention. Diagnostic testing methodology usually comprises a complete blood count in conjunction with one or more of hemoglobin electrophoresis, isoelectric focusing, high-performance liquid chromatography and DNA testing (22).

### Chronic anemia and hemolysis

The sickled erythrocyte has a shorter half-life than the normal erythrocyte and results from the instability of HbS and the effects of repeated episodes of hemoglobin polymerization/depolymerization in the circulation. This affects membrane ionic permeability, cellular viscosity and deformability (20) and promotes oxidative membrane damage (29). Sickle cell disease patients are anemic by 2 to 3 months of age and develop symptoms and complications associated with chronic anemia and hemolysis (22, 30) such as renal disease, ophthalmic disorders, leg ulcers, priapism and pulmonary hypertension (26, 31-37). Hemoglobin values for SCD patients range from 6 to 10g/dL and the hemoglobin S molecule has a poor affinity for oxygen. Triggers for transfusion in patients are a hemoglobin value of 5 or less or a precipitous drop in hemoglobin of 2g/dL or more. Transfusions are typically given to restore hemoglobin values to baseline levels established for each patient as excessive hematocrit can precipitate sickling (38). SCD patients are more susceptible to parvovirus B19 infection which can arrest erythropoiesis and lead to aplastic anemia crisis (39).

### Vasoocclusive pain crisis

Vascular occlusion is central to the clinical course of SCD and likely involves both the micro and macro circulation. Occlusion occurring in the microvasculature can culminate in acute painful episodes or vasoocclusive pain crises. Vasoocclusive pain crisis is the clinical hallmark of microvascular occlusions and accounts for over 90% of hospital admissions of adults SCD patients. It is well known that polymerization of hemoglobin S during deoxygenation and cell sickling leads to blockage of the microvasculature (40). However, it has recently become clear that hemoglobin S polymerization is not solely responsible for vasoocclusion. It has now been demonstrated that such events as sickled red cell lysis, cell membrane damage and oxidative stress, repeated ischemic damage, and microvasculature injury due to the adhesive interactions between sickle red cells and the endothelium that culminate in a proinflammatory environment (41-43). In this environment of chronic vascular inflammation, the adherence of leukocytes, platelets and sickled red cells to activated blood vessel endothelium and to each other is believed to be a primary cause of microvasculature blockage and vasoocclusive pain crisis (43-47). Additional factors such as the rigidity of sickled cells, increased blood viscosity, and local vasoconstriction have also been identified as potentially contributing to the vasoocclusion process.

Long-term repeated vasoocclusive events and occlusions occurring in the macrovasculature can cause life-threatening complications leading to organ damage and failure, stroke and death (40). There is an approximately 20 to 30 year reduction in life expectancy in sickle cell disease patients (48). Chronic pain in SCD is not just a continuation of the pain of vasoocclusion: it is usually secondary to avascular necrosis of bone at various joints (49). Sickled red cells can become trapped in the spleen causing it to become enlarged and precipitating splenic sequestration crisis causing sudden and severe anemia. Functional asplenia leaves patients susceptible to infection (18). Bone growth retardation, renal (32), ophthalmic (33) and cerebrovascular complications (ranging from clinically evident acute stroke to transient silent ischemic infarct) (50) are seen as major clinical consequences of SCD and vasoocculsive injury (22). Acute chest syndrome is another major complication (51), and is a significant cause of morbidity and mortality (52).

Pain episodes appear to be triggered by a number of factors including cold, stress and physical exertion (38, 53). The frequency, severity, location and duration of pain crises can vary considerably, even within a specific disease subtype. Patients with homozygous sickle cell and sickle cell β°-thalassemia have a higher frequency of vasoocclusive pain crises than patients with hemoglobin SC and sickle cell- β°-thalassemia genotype (54). Disease severity is thought to depend on a complex interaction of genetic, rheologic and hematologic factors, as well as microvascular and endothelial factors. Crises commonly involve pain in the back, legs, knees, arms, chest and abdomen (53). The frequency of crisis and pain severity varies considerably among patients and in the same patient over time. One study evaluating pain rates in patients ranging from newborns to age 50 years indicated that 5.2 percent of patients with sickle cell disease have three to 10 episodes of severe pain every year (54). In an independent study, over 30% of sickle cell patients in the US (approximately 27,000 patients) have three or more pain crises per year (55). Moreover, a recent study (PiSCES) evaluating health related quality of life issues in SCD patients indicated that pain crisis might be significantly underreported among SCD patients (56).

### Current Therapies For Vascular Occlusion

Vascular occlusion in SCD patients can manifest in multiple ways including vasoocclusive pain crisis, acute chest syndrome, cerebrovascular events and multiple types of organ failure. Therefore, treatment modalities for vascular occlusion depend on the clinical course and severity of the disease and are generally symptomatic or palliative in nature. Patient education in the avoidance of initiating factors that precipitate vasoocclusive pain crisis has shown some prophylactic benefit. The two most common symptomatic treatments are blood transfusions and analgesics. Most SCD patients commonly have hemoglobin values between 6 and 10g/dL and hemoglobin values typically drop at least 1 g per dL during a vasoocclusive pain crisis. Severe pain resulting from vasoocclusive crisis can be treated with narcotics but their use is controversial due to concerns of narcotic addiction and tolerance. Other complications with narcotic use are drug-seeking behavior, sedation and respiratory depression. Oxygen management has been utilized to treat vasoocclusive pain crisis despite the lack of strong evidence supporting its effectiveness. Rehydration is also sometime used during vasoocclusive pain crises with some benefit (22, 38).

Bone marrow transplantation may be considered and can be curative, but its use is restricted to a limited number of patients, and carries a high risk of morbidity and mortality (22).

Hydroxyurea (Droxia) is the only FDA approved drug for treatment of SCD pain crises. The mechanisms by which it produces its beneficial effects are uncertain but may involve increasing hemoglobin F levels in RBCs thereby decreasing the level of hemoglobin S polymerization. Hydroxyurea is cytotoxic, myelosuppressive and teratogenic (57, 58) which implies a carcinogenic risk to SCD patients. The long-term effects however, on hematologic toxicities, organ damage and carcinogenicity are currently unknown (59, 60).

In summary, most therapies for vasoocclusive pain crisis in SCD patients provide symptomatic relief and do not address the underlying cause of this debilitating condition. To date only one therapy has been approved by the FDA for the treatment of pain crisis, thus, patients with SCD represent a major unmet medical need in a life-threatening disease with severe morbidities.

### P-selectin as a Therapeutic Target for SCD

In SCD, as noted above, interactions between sickled red cells, platelets, leukocytes and the microvasculature are P-selectin-dependent processes and result in vasoocclusion and painful crisis. Studies in transgenic mice engineered to express human β hemoglobin S (β^{s}) have shown that antibody-mediated inhibition of P-selectin function can prevent and/or reduce vasoocclusion, indicating a therapeutic potential for this target. In addition mice expressing the β^{s} hemoglobin that lack P-selectin (due to gene deletion) do not suffer vasoocclusion, further supporting a key role for this molecule in this morbidity.

The hyper-inflammatory state in SCD patients is characterized by activated monocytes and vascular endothelium (61-63). A similar pro-inflammatory phenotype was demonstrated in resting state β^{s} mice which exhibit elevated levels of peripheral leukocytes and neutrophils, an increased number of rolling and adherent leukocytes, and reduced blood flow volume and red blood cell velocities (64). The β^{s} mice were hypersensitive to hypoxia/reoxygenation resulting in an inflammatory response represented by a significant increase in the number of adherent and emigrated leukocytes. This inflammatory response was completely blocked by a functionally blocking anti-mouse P-selectin antibody, but not by a functionally blocking anti-mouse E-selectin antibody, demonstrating a critical role for P-selectin in this process.

P-selectin plays its central role in the recruitment of leukocytes to inflammatory and thrombotic sites by binding to its counter-receptor, P-selectin glycoprotein ligand-1 (PSGL-1) (or a PSGL-1-like receptor on sickled red blood cells), which is a mucin-like glycoprotein constitutively expressed on leukocytes including neutrophils, monocytes, platelets, and on some endothelial cells (68). The ultimate physiologic function of the selectins is to promote extravasation of leukocytes into inflamed or damaged tissues. The initial binding of P-selectin on the endothelium to PSGL-1 on the leukocytes is essential and central to this process. The predominant mechanism for rolling and tethering of leukocytes to activated endothelium and platelets is the binding of leukocyte PSGL-1 to the P-selectin on these cells (68, 69). PSGL-1 binds to P-, L- and E-selectin (70). P-selectin and SGP-3, a glycosulfopeptide modeled from the N-terminus of PSGL-1, have been co-crystallized and the contact residues for lectin-ligand binding have been identified (71).

The selectins share common structural motifs including a lectin domain (or carbohydrate recognition domain), an epidermal growth factor-like domain (EGF), a varying series of consensus repeats, a transmembrane domain and a cytoplasmic tail (70). As noted, the initial tethering and rolling of leukocytes is mediated by the interaction of P-selectin and PSGL-1. Thus the blocking of P-selectin function by using (1) antibodies to P-selectin, (2) antibodies to PSGL-1, (3) fragments of PSGL-1 or recombinant forms of PSGL-1, (4) small molecules that mimic the binding domain of PSGL-1, and (5) other molecules that disrupt the binding of P-selectin to PSGL-1, can block leukocyte rolling and tethering and thus prevent firm adhesion to endothelial cells or platelets. Mice deficient in P-selectin or PSGL-1 also fail to support leukocyte tethering and rolling on activated endothelial cells (72, 74). L-selectin plays a dual role in that it is constitutively expressed on circulating leukocytes and can initiate "secondary binding" by interaction with PSGL-1 on other leukocytes (75). This process leads to further recruitment of new leukocytes to the inflamed area. L-selectin binding to PSGL-1 also plays a role in homing of lymphocytes to the high endothelial vasculature (HEV) venules in the secondary lymphatic system (76). E-selectin is transcriptionally regulated and is expressed on activated endothelial cells hours after P-selectin mediated events. E-selectin can bind PSGL-1 with low affinity but can also bind other ligands. Single transgenic knockout mice for each selectin have shown that these molecules possess compensatory selectin mechanisms for leukocyte homing and rolling (77).

In view of the above, there is a well-established need for new treatments, such as antibodies, that target P-selectin as a means of treating inflammatory and thrombotic diseases by disrupting the binding of P-selectin and PSGL-1. It is therefore a goal of the presently disclosed inventive concepts to block P-selectin binding to PSGL-1 thereby blocking the adherence of blood cells that contribute to vasoocclusion in SCD and other thrombotic disorders as discussed elsewhere herein.

### SUMMARY OF THE DISCLOSURE

The presently disclosed inventive concepts are directed to "dual function" antibodies which bind specifically to P-selectin and which not only block the binding of PSGL-1 to P-selectin, but also dissociate preformed P-selectin/PSGL-1 complexes. The present disclosure describes a heretofore unrecognized antibody binding domain (a conformational epitope) within the lectin domain (e.g., carbohydrate recognition domain, CRD) of P-selectin to which the dual function antibodies (which may be chimeric, human or humanized antibodies or fragments thereof for example) bind. The presently disclosed inventive concepts are therefore directed to anti-P-selectin antibodies or fragments thereof which bind to the conformational epitope described herein and which have a dual function in (1) blocking binding of PSGL-1 to P-selectin, and (2) causing dissociation of preformed P-selectin/PSGL-1 complexes. The presently disclosed inventive concepts in particular are directed to using such dual function anti-P-selectin antibodies or antibody fragments as described herein in treatments for inflammatory, thrombotic or other conditions or disorders in primates (including humans) which involve platelet, sickled red cell, leukocyte, lymphocyte, and/or endothelial cell adhesion, wherein the condition or disorder comprises or is associated with (but not limited to) at least one of sickle cell vasoocclusive pain crisis, inflammatory bowel disease (e.g., Crohn's Disease, ulcerative colitis, and enteritis), arthritis (e.g., rheumatoid arthritis, osteoarthritis, and psoriatic arthritis), graft rejection, graft versus host disease, asthma, chronic obstructive pulmonary disease, psoriasis, dermatitis, sepsis, nephritis, lupus erythematosis, scleroderma, rhinitis, anaphylaxis, diabetes, multiple sclerosis, atherosclerosis, thrombosis, tumor metastasis, allergic reactions, thyroiditis, ischemic reperfusion injury (e.g., due to myocardial infarction, stroke, or organ transplantation), and conditions associated with extensive trauma, or chronic inflammation, such as, for example, type IV delayed hypersensitivity, associated for example with infection by Tubercle bacilli, or systematic inflammatory response syndrome, or multiple organ failure. Importantly, the use of such dual function antibodies as described herein in treating these inflammatory diseases allow not only the prevention of inflammation, but also provide a mechanism to treat ongoing inflammatory disease processes in that the antibodies can dissociate preformed P-selectin/PSGL-1. For example, in the case of sickle cell vasoocclusive pain crisis, antibodies having dual function activity not only inhibit or prevent future vasoocclusive events, but also allow the treatment of ongoing vasoocclusion. Other embodiments of the inventive concepts disclosed herein will be apparent in the Detailed Description below.

### BRIEF DESCRIPTION OF THE DRAWINGS

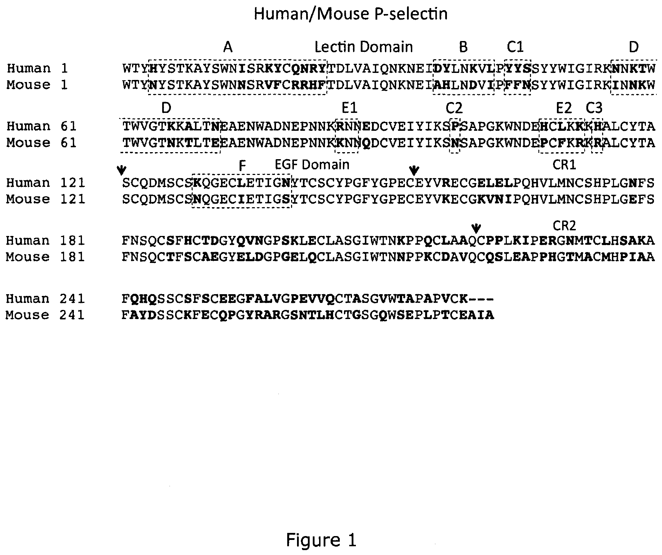

Figure 1 shows a homology comparison at the amino acid level of human and mouse P-selectin indicating the location of lectin, EGF, CR1 and CR2 domains (transition between domains is indicated by arrows ↓). Nonlinear conformational domains A, B, C1, D, E1, C2, E2, C3, and F are indicated by dashed boxes. Amino acid differences are indicated in boldface.

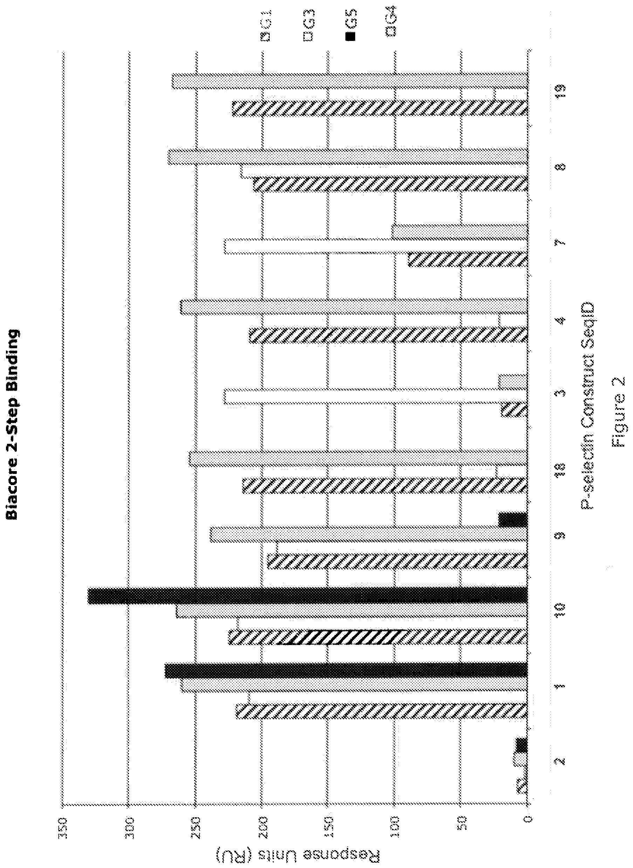

Figure 2 shows representative two-step BIACORE P-selectin chimera binding data for the anti-P-selectin antibodies G1, G3, G4 and G5 binding to SEQ ID NO:1-4, 7-10, 18 and 19. Note that binding of the G5 antibody in the Biacore two-step binding assay was only performed for SEQ ID NOs: 1, 2, 18 and 19. G4 is a novel anti-P-selectin mouse monoclonal antibody which is produced by hybridoma cells deposited as Patent Deposit Designation PTA-12154 in the American Type Culture Collection, 10801 University Blvd., Manassas, VA 20110-2209, a recognized public depository.

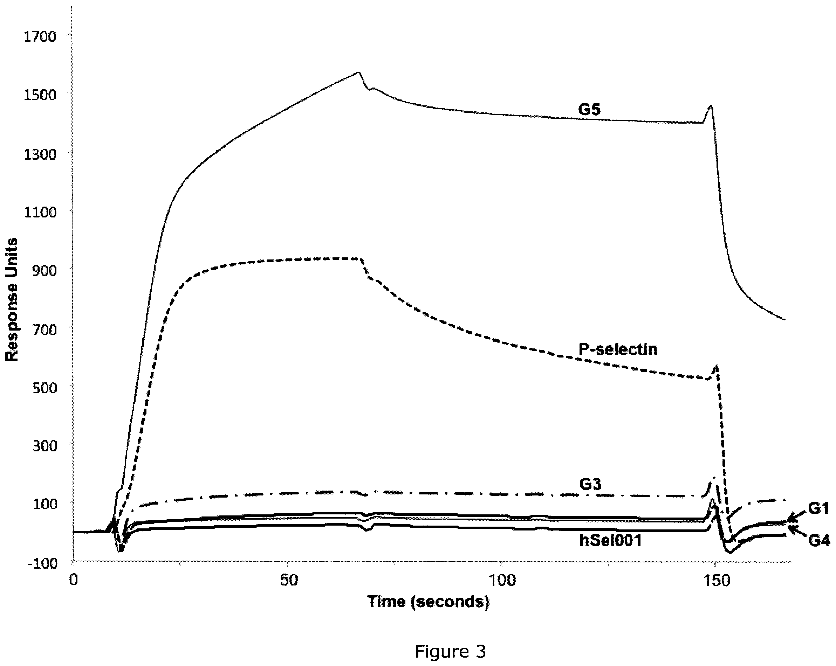

Figure 3 shows BIACORE sensograms demonstrating blocking of P-selectin interaction with PSGL-1 using anti-P-selectin antibodies G1, G3, G4 and hSel001 (hSel001, also known as SelG1, is a humanized form of G1) by the methods described herein. G1, G3, G4 and hSel001 were shown to block interaction of P-selectin to the glycosulfopeptide GSP-6, a PSGL-1 mimetic. G5 binds P-selectin, but did not block. The control is steady state binding of P-selectin to GSP-6.

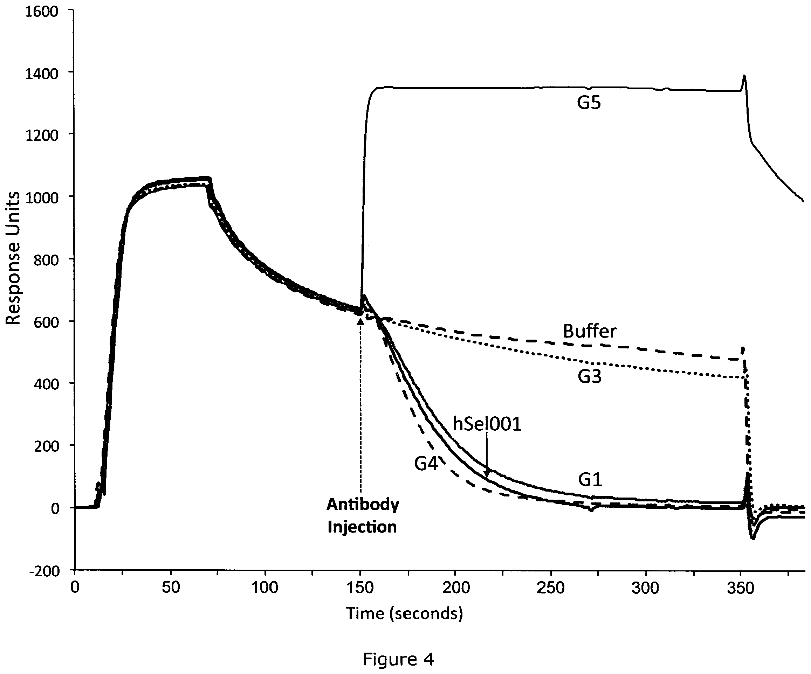

Figure 4 shows BIACORE sensograms demonstrating dissociation of the preformed P-selectin/PSGL-1 complex upon exposure to dual function anti-P-selectin antibodies G1, G4 and hSel001. PSGL-1 is represented by GSP-6 peptide, a PSGL-1 mimetic. Initial RU increase shows binding of P-selectin to biotin-GSP-6 coupled to a streptavidin coated BIACORE chip. Once steady state binding of the P-selectin/GSP-6 complex was reached (i.e., after normal dissociation of the complex had reached near-equilibrium), test antibodies were injected and assessed for dissociation properties. G5 bound to the preformed complex, but did not cause its dissociation. G3 did not bind or dissociate the preformed P-selectin/PSGL-1 complex. G1, G4 and hSel001 both bound and caused dissociation of the preformed P-selectin/GSP-6 complex, indicating novel dual function capabilities.

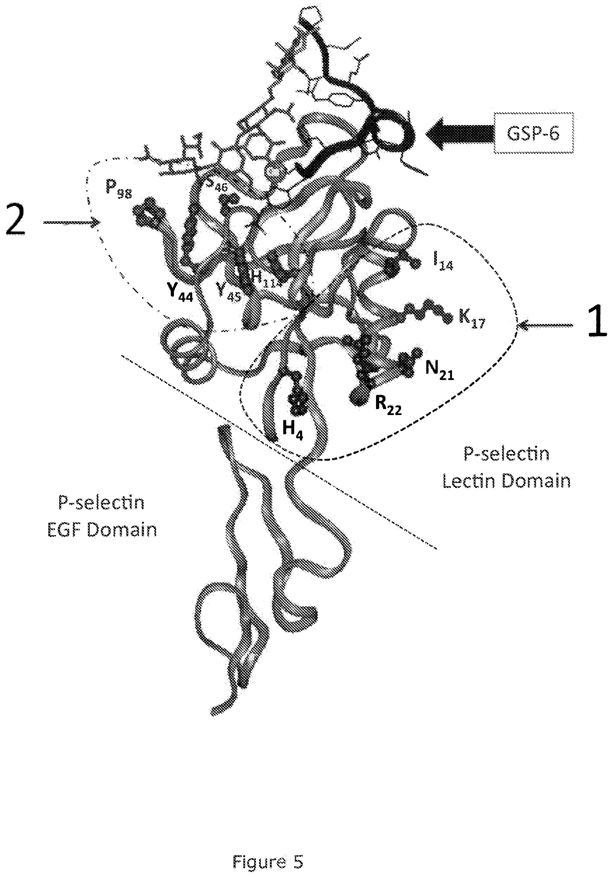

Figure 5 shows a 3-D representation of a human P-selectin molecule with GSP-6 binding thereto. Lectin and EGF domains are demarcated by a dashed line. Binding region 1 indentifies a Cluster A conformational epitope that is distal to the lectin/ligand binding domain. Test antibody G1 bound to region 1, Cluster A. G4 and hSel001 also bound region 1, Cluster A.

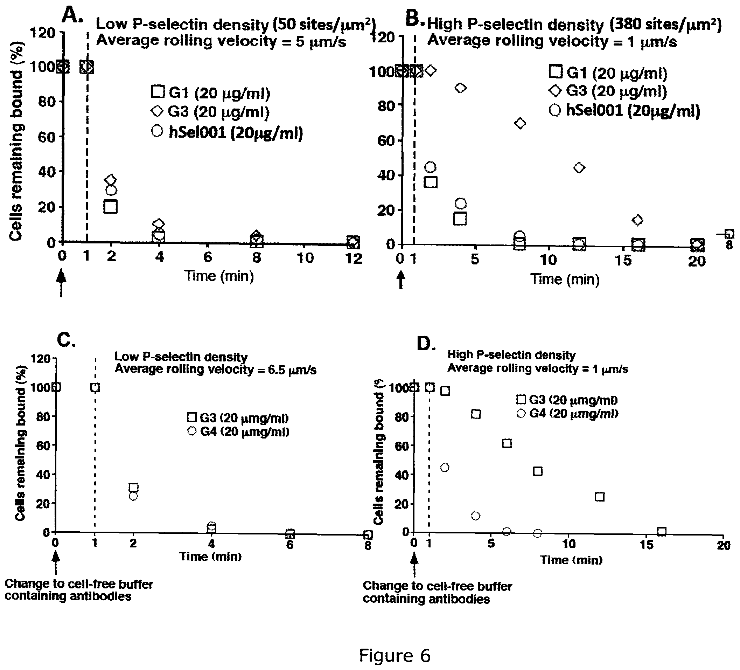

Figure 6 shows graphs of results of cell-based *in vitro* rolling assays under flow of human neutrophils on low and high density P-selectin. Results demonstrate blocking and/or dissociation of the preformed P-selectin/PSGL-1 complex and subsequent release of neutrophils upon exposure to antibodies G1, G3, G4 and hSel001. Antibodies were introduced at equivalent concentrations of 20µg/ml for the duration of the study. There is a lag time of about 1 minute before the antibody reaches the chamber due to the dead volume of the system. At 1-minute intervals thereafter, cells remaining bound were counted and expressed as % cells bound. Panels (A) and (C) show neutrophils rolling at average velocities of 5µm/s and 6.5µm/s respectively on low density (50 sites /µm²) membrane P-selectin. Panels (B) and (D) show neutrophils rolling at an average velocity of 1µm/s on high density P-selectin (380 sites/µm²).

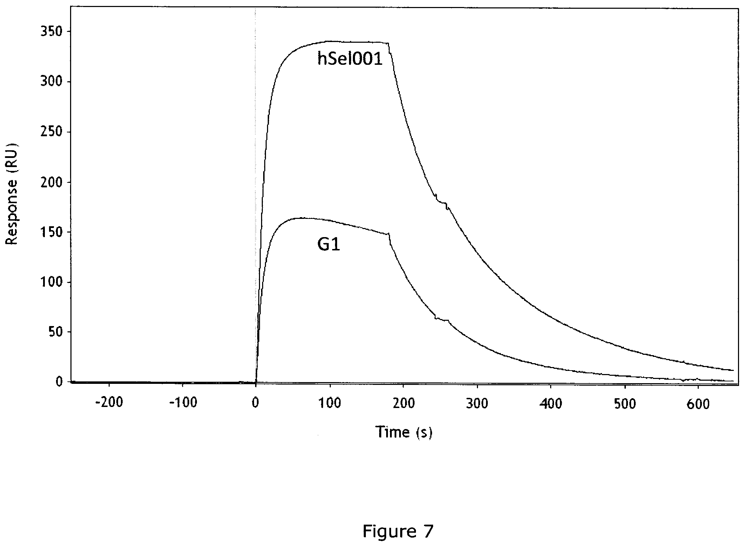

Figure 7 is a sensogram showing kinetics for G1 and hSel001 binding to P-selectin at a single P-selectin concentration.

### DETAILED DESCRIPTION

Unless otherwise defined herein, scientific and technical terms used in connection with the presently disclosed inventive concepts shall have the meanings that are commonly understood by those of ordinary skill in the art. Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those well known and commonly used in the art. Standard techniques are used for recombinant DNA, oligonucleotide synthesis, and tissue culture and transformation (e.g., electroporation, lipofection). Enzymatic reactions and purification techniques are performed according to manufacturer's specifications or as commonly accomplished in the art or as described herein. The foregoing techniques and procedures are generally performed according to conventional methods well known in the art and as described in various general and more specific references that are cited and discussed throughout the present specification. See e.g., Sambrook et al. Molecular Cloning: A Laboratory Manual (2nd ed., Cold Spring Harbor Laboratory Press, Cold Spring Harbor, N.Y. (1989) and Coligan et al. Current Protocols in Immunology (Current Protocols, Wiley Interscience (1994)). The nomenclatures utilized in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

As utilized in accordance with the present disclosure, the following terms, unless otherwise indicated, shall be understood to have the following meanings:

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects. The use of the term "at least one" will be understood to include one as well as any quantity more than one, including but not limited to, 2, 3, 4, 5, 10, 15, 20, 30, 40, 50, 100, etc. The term "at least one" may extend up to 100 or 1000 or more, depending on the term to which it is attached; in addition, the quantities of 100/1000 are not to be considered limiting, as higher limits may also produce satisfactory results.

The term "about" is used to indicate that a value includes the inherent variation of error for the device, the method being employed to determine the value and/or the variation that exists among study subjects.

As used in this specification and claims, the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, MB, BBC, AAABCCCC, CBBAAA, CABABB, and so forth. The skilled artisan will understand that typically there is no limit on the number of items or terms in any combination, unless otherwise apparent from the context.

The terms "peptide", "polypeptide" and "protein" are used herein to refer to a polymer of amino acid residues. The term "polypeptide" as used herein is a generic term to refer to native protein, protein fragments, or analogs of a polypeptide sequence. Hence, native protein, protein fragments, and analogs are species of the polypeptide genus. The term "isolated peptide/polypeptide/protein" as used herein refers to a peptide/polypeptide/protein of cDNA, recombinant RNA, or synthetic origin or some combination thereof, which by virtue of its origin, or source of derivation, the "isolated peptide/polypeptide/protein": (1) is not associated with peptides/polypeptides/proteins found in nature, (2) is free of other peptides/polypeptides/proteins from the same source, e.g., free of murine proteins, (3) is expressed by a cell from a different species, and/or (4) does not occur in nature.

As used herein, the term "amino acid" embraces all molecules, whether natural or synthetic, which include both an amino functionality and an acid functionality and capable of being included in a polymer of naturally-occurring amino acids. Exemplary amino acids include naturally-occurring amino acids; analogs, derivatives and congeners thereof; amino acid analogs having variant side chains; and all stereoisomers of any of any of the foregoing. Where used herein the term "mouse amino acid" refers to an amino acid residue which is found in mouse P-selectin but is not found in the corresponding position in human P-selectin. Where used herein the term "human amino acid" refers to an amino acid residue which is found in human P-selectin but is not found in the corresponding position in mouse P-selectin.

As used herein, the twenty conventional amino acids and their abbreviations follow conventional usage. See Immunology--A Synthesis (2nd Edition, E. S. Golub and D. R. Gren, Eds., Sinauer Associates, Sunderland, Mass. (1991)). Stereoisomers (e.g., D-amino acids) of the twenty conventional amino acids, unnatural amino acids such as α,α-disubstituted amino acids, N-alkyl amino acids, lactic acid, and other unconventional amino acids may also be suitable components for polypeptides of the presently disclosed and claimed inventive concept(s). Examples of unconventional amino acids include: 4-hydroxyproline, α-carboxyglutamate, ε-N,N,N-trimethyllysine, ε-N-acetyllysine, O-phosphoserine, N-acetylserine, N-formylmethionine, 3-methylhistidine, 5-hydroxylysine, σ-N-methylarginine, and other similar amino acids and imino acids (e.g., 4-hydroxyproline). In the polypeptide notation used herein, the lefthand direction is the amino terminal direction and the righthand direction is the carboxy-terminal direction, in accordance with standard usage and convention.

The terms "polynucleotide", and "nucleic acid" are used interchangeably. They refer to a polymeric form of nucleotides of any length, either deoxyribonucleotides or ribonucleotides, or analogs thereof. The following are nonlimiting examples of polynucleotides: coding or non-coding regions of a gene or gene fragment, loci (locus) defined from linkage analysis, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified, such as by conjugation with a labeling component. The terms "isolated nucleic acid" and "isolated polynucleotide" are used interchangeably; a nucleic acid or polynucleotide is considered "isolated" if it: (1) is not associated with all or a portion of a polynucleotide in which the "isolated polynucleotide" is found in nature, (2) is linked to a polynucleotide to which it is not linked in nature, or (3) does not occur in nature as part of a larger sequence.

The term "vector," as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby be replicated along with the host genome. Moreover, certain vectors are capable of directing the expression of genes. Such vectors are referred to herein as "recombinant expression vectors" (or simply, "expression vectors").

The term "naturally-occurring" as used herein as applied to an object refers to the fact that an object can be found in nature. For example, a polynucleotide or polypeptide sequence that is present in an organism (including viruses) that can be isolated from a source in nature and which has not been intentionally modified by man in the laboratory or otherwise is naturally-occurring. The term "naturally-occurring" may be used interchangeably herein with the term "native".

"Leukocyte rolling," as used herein, includes weak adhesion of leukocytes to endothelial cells of blood vessels and rolling of leukocytes along endothelial cells of blood vessels prior to firm adhesion and transmigration of leukocytes into endothelial tissue. Following leukocyte rolling, these adherent leukocytes can migrate through the endothelium and destroy ischemic tissue during reperfusion. Accordingly, reduction of leukocyte rolling results in a reduction of damage to tissues and organs caused by acute inflammatory responses.

As used herein, a "P-selectin antagonist" includes any agent which is capable of antagonizing P-selectin, e.g., by inhibiting interaction between P-selectin and a P-selectin glycoprotein ligand-1, e.g., by inhibiting interactions of P-selectin expressing endothelial cells and activated platelets with PSGL-1 expressing leukocytes.

The term "isolated" or "purified" refers to a molecule that is substantially free of its natural environment and is the predominant species present (e.g., on a molar basis) such as more than 50% of the composition. For instance, an isolated protein is substantially free of cellular material or other proteins from the cell or tissue source from which it was derived. The term also refers to preparations where the isolated protein is at least 60% (w/w) pure, or at least 70% (w/w) pure; or at least 75% (w/w) pure; or at least 80% (w/w) pure; or at least 85% (w/w) pure, or at least 90% (w/w) pure, or at least 92% (w/w) pure, or at least 95% (w/w) pure, or at least 96% (w/w) pure, or at least 97% (w/w) pure, or at least 98% (w/w) pure, or at least 99% (w/w) pure, or 100% (w/w) pure. In some embodiments, the isolated molecule is sufficiently pure for pharmaceutical compositions.

"Inhibitory" activity refers to a reduction in an activity of P-selectin by a P-selectin inhibitor (such as an antibody or fragment thereof), relative to the activity of P-selectin in the absence of the same inhibitor. A neutralizing antibody may reduce one or more P-selectin activities. For example, the reduction in activity (e.g., P-selectin binding to PSGL-1) is preferably at least about 10%, at least 20%, at least 30%, at least 40%, at least 50%, at least 60%, at least 70%, at least 75%, at least 80%, at least 85%, at least 90%, or at least 95%, or higher. In another example, the dissociative activity of a dual function antibody or fragment (i.e., the percentage of preformed P-selectin/PSGL-1 complex which may be caused to dissociate) may be at least 10%, at least 20%, at least 30%, at least 40%, at least 50%, at least 60%, at least 70%, at least 75%, at least 80%, at least 85%, at least 90%, at least 95%, or higher.

The term "P-selectin inhibitor" when used herein includes any agent, such as, e.g., a neutralizing antibody, capable of inhibiting activity, expression, processing, binding, or cell surface localization of P-selectin. Such inhibitors are said to "inhibit," "neutralize," or "reduce" the biological activity of P-selectin.

The term "effective amount" refers to an amount of a biologically active molecule or conjugate or derivative thereof sufficient to exhibit a detectable therapeutic effect preferably without undue adverse side effects (such as toxicity, irritation and allergic response) commensurate with a reasonable benefit/risk ratio when used in the manner of the presently disclosed inventive concepts. The term "pharmaceutically acceptable" refers to compounds and compositions which are suitable for administration to humans and/or animals without undue adverse side effects such as toxicity, irritation and/or allergic response commensurate with a reasonable benefit/risk ratio. The compounds of the presently disclosed inventive concepts may be designed to provide delayed, controlled or sustained release using formulation techniques which are well known in the art.

The term "epitope" refers to an antigenic determinant in a polypeptide that interacts with a specific antigen binding site in the variable region of an antibody molecule known as a paratope. Epitopes may be either linear or conformational. A conformational epitope is produced by spatially juxtaposed amino acids from different segments of a linear polypeptide chain.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, generally being directed against a single epitopic site. Furthermore, in contrast to conventional polyclonal antibody preparations that typically include different antibodies directed against different determinants (epitopes), each monoclonal antibody is directed against a single determinant. In addition to their specificity, the monoclonal antibodies are advantageous in that, in one embodiment, they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method.

### Antibodies

Antibody molecules belong to a family of plasma proteins called immunoglobulins, whose basic building block, the immunoglobulin fold or domain, is used in various forms in many molecules of the immune system and other biological recognition systems. A typical immunoglobulin has four polypeptide chains, containing an antigen binding region known as a variable region and a non-varying region known as the constant region.

Native antibodies and immunoglobulins are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain at one end (VL) and a constant domain at its other end. The constant domain of the light chain is aligned with the first constant domain of the heavy chain, and the light chain variable domain is aligned with the variable domain of the heavy chain.

Depending on the amino acid sequences of the constant domain of their heavy chains, immunoglobulins can be assigned to different classes. There are at least five major classes of immunoglobulins: IgA, IgD, IgE, IgG and IgM, and several of these may be further divided into subclasses (isotypes), e.g. IgG₁, IgG₂, IgG₃ and IgG₄ and IgA₁ and IgA₂. The constant domains of the heavy chains that correspond to the different classes of immunoglobulins are called alpha (α), delta (δ), epsilon (ε), gamma (γ) and mu (µ), respectively. The light chains of antibodies can be assigned to one of two clearly distinct types, called kappa (κ) and lambda (λ), based on the amino sequences of their constant domain. The subunit structures and three-dimensional configurations of different classes of immunoglobulins are well known.

The term "variable" in the context of variable domain of antibodies, refers to the fact that certain portions of the variable domains differ extensively in sequence among antibodies. The variable domains are for binding and determine the specificity of each particular antibody for its particular antigen. However, the variability is not evenly distributed through the variable domains of antibodies. It is concentrated in three segments per chain called complementarity determining regions (CDRs), also known as hypervariable regions, both in the light chain and the heavy chain variable domains. In most instances, three CDRs are present in a light chain variable region (CDRL1, CDRL2 and CDRL3) and three CDRs are present in a heavy chain variable region (CDRH1, CDRH2 and CDRH3). CDRs contribute to the functional activity of an antibody molecule and are separated by amino acid sequences that comprise scaffolding or framework regions. Among the various CDRs, the CDR3 sequences, and particularly CDRH3, are the most diverse and therefore have the strongest contribution to antibody specificity. There are at least two techniques for determining CDRs: (1) an approach based on cross-species sequence variability (i.e., Kabat et al., Sequences of Proteins of Immunological Interest (National Institute of Health, Bethesda, Md. (1987)); and (2) an approach based on crystallographic studies of antigen-antibody complexes (Chothia et al., Nature, 342:877 (1989), incorporated by reference in its entirety).

The more highly conserved portions of variable domains are called the framework (FR). The variable domains of native heavy and light chains each comprise four FR regions, largely adopting a β-sheet configuration, connected by three CDRs, which form loops connecting, and in some cases forming part of, the β-sheet structure. The CDRs in each light and heavy chain are held together in close proximity by the FR regions and contribute to the formation of the antigen-binding site of the antibody. The constant domains are not involved directly in binding an antibody to an antigen, but exhibit various effector functions, such as participation of the antibody in antibody-dependent cellular toxicity.

An antibody of the presently disclosed inventive concepts thus can be in any of a variety of forms, including a whole immunoglobulin, an antibody fragment such as Fv, Fab, and similar fragments, a single chain antibody which includes the variable domain complementarity determining regions (CDRs), and the like forms, all of which fall under the broad term "antibody", as used herein. In preferred embodiments, in the context of both the therapeutic and screening methods described below, an antibody or fragment thereof is used that is immuno-specific for an antigen or epitope of the presently disclosed inventive concepts as described herein.

The term "antibody fragment" as used herein refers to a portion of a full-length antibody, generally the antigen binding or variable region. Examples of antibody fragments include Fab, Fab', F(ab')₂ and Fv fragments. Papain digestion of antibodies produces two identical antigen binding fragments, called the Fab fragment, each with a single antigen binding site, and a residual "Fc" fragment, so-called for its ability to crystallize readily. Pepsin treatment yields an F(ab')₂ fragment that has two antigen binding fragments that are capable of cross-linking antigen, and a residual other fragment (which is termed pFc'). Additional fragments can include diabodies, linear antibodies, single-chain antibody molecules, and multispecific antibodies formed from anti-body fragments. As used herein, "functional fragment" with respect to antibodies, refers to Fv, F(ab) and F(ab')₂ fragments. Fragments of the antibodies of the presently disclosed inventive concepts may be as small as about 4, 5, 6, 7, 8, 9, 10, 11, 12, 13, 14, 15, 16, 17, 18, 19, 20, 21, 22, 23, 24, 25, 26, 27, 28, 29, or 30 to 35, to 40, to 45, to 50, to 75, to 100, or to 150 to 200 to 250 (all inclusive) or more amino acids, for example.

Some types of antibody fragments are defined as follows:
Fab is the fragment that contains a monovalent antigen-binding fragment of an antibody molecule. A Fab fragment can be produced by digestion of whole antibody with the enzyme papain to yield an intact light chain and a portion of one heavy chain.
Fab' is the fragment of an antibody molecule can be obtained by treating whole antibody with pepsin, followed by reduction, to yield an intact light chain and a portion of the heavy chain. Two Fab' fragments are obtained per antibody molecule.
Fab' fragments differ from Fab fragments by the addition of a few residues at the carboxyl terminus of the heavy chain CH1 domain including one or more cysteines from the antibody hinge region.
(Fab')₂ is the fragment of an antibody that can be obtained by treating whole antibody with the enzyme pepsin without subsequent reduction. F(ab')₂ is a dimer of two Fab' fragments held together by two disulfide bonds.
Fv is the minimum antibody fragment that contains a complete antigen recognition and binding site. This region consists of a dimer of one heavy and one light chain variable domain in a tight, non-covalent association (VH-VL dimer). It is in this configuration that the three CDRs of each variable domain interact to define an antigen binding site on the surface of the VH-VL dimer. Collectively, the six CDRs confer antigen binding specificity to the antibody. However, even a single variable domain (or half of an Fv comprising only three CDRs specific for an antigen) has the ability to recognize and bind antigen, although at a lower affinity than the entire binding site.

A single chain antibody (SCA) is defined herein as a genetically engineered molecule containing the variable region of the light chain, the variable region of the heavy chain, linked by a suitable polypeptide linker as a genetically fused single chain molecule. Such single chain antibodies are also referred to as "single-chain Fv" or "sFv" or "scFv" antibody fragments. Generally, the Fv polypeptide further comprises a polypeptide linker between the VH and VL domains that enables the sFv to form the desired structure for antigen binding.

The application discloses antibodies that specifically bind to human P-selectin. CDRs in such antibodies are not limited to the specific sequences of VH and VL and may include variants of these sequences that retain the ability to block and dissociate P-selectin binding to PSGL-1. Such variants may be produced by a skilled artisan using techniques well known in the art. For example, amino acid substitutions, deletions, or additions, can be made in the FRs and/or in the CDRs as described elsewhere herein. While changes in the FRs are usually designed to improve stability and decrease immunogenicity of the antibody, changes in the CDRs are typically designed to increase affinity of the antibody for its target. Variants of FRs also include naturally occurring immunoglobulin allotypes. Such affinity-increasing changes may be determined empirically by routine techniques that involve altering the CDR and testing the affinity antibody for its target.

For example, conservative amino acid substitutions can be made within any one of the disclosed CDRs. Various alterations can be made according to methods well known to those skilled in the art (78). These include but are not limited to nucleotide sequences that are altered by the substitution of different codons that encode an identical or a functionally equivalent amino acid residue ("conservative substitutions") within the sequence, thus producing a "silent" change. For example, the nonpolar amino acids which may be conservatively substituted for each other include alanine, leucine, isoleucine, valine, proline, phenylalanine, tryptophan, and methionine. The polar neutral amino acids which may be conservatively substituted for each other include glycine, serine, threonine, cysteine, tyrosine, asparagine, and glutamine. The positively charged (basic) amino acids which may be conservatively substituted for each other include arginine, lysine, and histidine. The negatively charged (acidic) amino acids which may be conservatively substituted for each other include aspartic acid and glutamic acid. Substitutes for an amino acid within the sequence may also be selected from other members of the class to which the amino acid belongs.

Derivatives and analogs of antibodies of the presently disclosed inventive concepts can be produced by various techniques well known in the art, including recombinant and synthetic methods (79, 80). Antibodies in which CDR sequences differ only insubstantially and conservatively from those of the variable regions of anti-P-selectin antibodies such as hSel001, discussed in further detail below, are also encompassed within the scope of this invention. As noted above, typically, an amino acid is substituted by a related amino acid having similar charge, hydrophobic, or stereochemical characteristics. Such substitutions would be within the ordinary skills of an artisan. Further, a skilled artisan would appreciate that changes can be made in FRs without adversely affecting the binding properties of an antibody. Changes to FRs include, but are not limited to, humanizing a non-human derived or engineering certain framework residues that are important for antigen contact or for stabilizing the binding site, e.g., changing the class or subclass of the constant region, changing specific amino acid residues which might alter the effector function such as Fc receptor binding.

The presently disclosed inventive concepts in one embodiment are directed to antibodies that specifically bind to P-selectin wherein the CDRs of a parental nonhuman antibody are grafted into FRs of human acceptor antibodies, a process called antibody humanization. The humanization process is designed to reduce the immunogenicity of a nonhuman antibody while maintaining as much of the original affinity as possible. In one embodiment, the human heavy and/or light chain acceptor FRs are unmutated amino acid sequences of the germline sequences from which they were derived. Such germline FRs would be expected to be nonimmunogenic considering that these sequences are present in all humans prior to antibody rearrangement and affinity maturation. Furthermore, amino acid residues of such antibody FRs, particularly residues adjacent to or positioned near the CDRs, may require amino acid substitutions to better preserve the antibody binding affinity. For example, when key amino acids differ between a parental murine monoclonal antibody variable FR and the human variable FR acceptor, the human FR amino acids may be substituted by the mouse amino acid residues at those positions. Nonetheless, it is anticipated that the FRs of the heavy and/or light chain may contain no amino acid substitutions and antibodies derived from such humanization may still possess much of if not all the binding affinity of the parental antibody. Further, antibodies of the invention may contain human VH and/or VL FRs that are of purely germline sequence and also lack substitutions in one or both of these FR sequences.

As used herein, the "affinity" of the antibody for P-selectin or the conformational epitope thereof is characterized by its K_{d}, or dissociation constant. A stronger affinity is represented by a lower K_{d} while a weaker affinity is represented by a higher K_{d}. As such, an antibody of the present invention preferably has an affinity for a P-selectin conformational epitope represented by a K_{d} ≤ 1000nM, or ≤ 500nM, or ≤ 100nM, or ≤ 50nM, or more preferably by a K_{d} ≤ 25nM, and still more preferably by a K_{d} ≤ 10nM, and even more preferably by a K_{d} ≤ 5nM, or ≤ 1nM, or ≤ 0.1nM.

An antibody or antibody fragment "homolog," as used herein, means that a relevant amino acid sequence (preferably for example in the CDRs and/or variable domains VH and/or VL) of a protein or a peptide is at least 50% identical, at least 60% identical, at least 70% identical, at least 75% identical, at least 80% identical, at least 85% identical, at least 90% identical, at least 91% identical, at least 92% identical, at least 93% identical, at least 94% identical, at least 95% identical, at least 96% identical, at least 97% identical, at least 98% identical, at least 99% identical, at least 99.5% identical, or 100% identical to a given sequence. By way of example, such sequences may be variants derived from various species, or the homologous sequence may be recombinantly produced. The sequence may be derived from the given sequence by truncation, deletion, amino acid substitution, or addition. Percent identity between two amino acid sequences is determined by standard alignment algorithms such as, for example, Basic Local Alignment Tool (BLAST) and other alignment algorithms and methods of the art (81-84).

The preparation of monoclonal antibodies is conventional and well known to persons of ordinary skill in the art. Monoclonal antibodies can be isolated and purified from hybridoma cultures by a variety of well-established techniques. Such isolation techniques include affinity chromatography with Protein-A Sepharose, size-exclusion chromatography, and ion-exchange chromatography. In an alternative to preparing monoclonal antibody-secreting hybridomas, a monoclonal anti-P-selectin antibody can be identified and isolated by screening a recombinant combinatorial immunoglobulin library (e.g., an antibody phage display library) with the conformational epitopes described herein respectively to thereby isolate immunoglobulin library members that bind P-selectin in accordance with the present invention. Kits for generating and screening phage display libraries are commercially available (e.g., the Pharmacia Recombinant Phage Antibody System, Catalog No. 27-9400-01; and the Stratagene SurJZAP™. Phage Display Kit, Catalog No. 240612).

Methods of *in vitro* and *in vivo* manipulation of monoclonal antibodies are well known to those skilled in the art. For example, the monoclonal antibodies to be used in accordance with the present invention may be made by the hybridoma method first described by Kohler and Milstein (85), or may be made by recombinant methods, e.g., as described in U.S. Pat. No. 4,816,567, for example.

Another method involves humanizing a monoclonal antibody by recombinant means to generate antibodies containing, for example, human or primate specific and recognizable sequences.

Methods of making antibodies of the presently disclosed inventive concepts which bind with high affinity to human P-selectin or to the conformational epitopes thereof as described herein may comprise transfecting a cell with a DNA construct, the construct comprising a DNA sequence encoding at least a portion of the neutralizing P-selectin specific antibodies of the invention, culturing the cell under conditions such that the antibody protein is expressed by the cell, and isolating the antibody protein.

Preferably, the constant region has been modified to modulate (i.e. reduce or enhance) effector function as noted elsewhere as compared to the effector function of a wild-type immunoglobulin heavy chain Fc region. In various embodiments, the IgG constant region has reduced effector function, or alternatively it has increased effector fuction. Fc effector function includes, for example, antibody-dependent cellular cytotoxicity (ADCC), phagocytosis, complement-dependent cytotoxicity, and half-life or clearance rate function. The IgG amino acid sequence of the Fc domain can be altered to affect binding to Fc gamma receptors (and thus ADCC or phagocytosis functions), or to alter interaction with the complement system (complement-dependent cytotoxicity function).

In one embodiment, the antibody comprises a constant region or Fc portion that has low or no affinity for at least one Fc receptor. In an alternative embodiment, the second polypeptide has low or no affinity for complement protein C1q. In general, an effector function of an antibody can be altered by altering the affinity of the antibody for an effector molecule such as an Fc receptor. Binding affinity will generally be varied by modifying the effector molecule binding site. Disclosure of IgG modifications that alter interaction with effector molecules such as Fc receptors can be found for example in U.S. Patent Nos. 5,624,821 and 5,648,260.

Antibody proteins of the presently disclosed inventive concepts can be produced using techniques well known in the art. For example, the antibody proteins can be produced recombinantly in cells (79, 86).

For recombinant production, a polynucleotide sequence encoding the antibody protein is inserted into an appropriate expression vehicle, such as a vector which contains the necessary elements for the transcription and translation of the inserted coding sequence. The expression vehicle is then transfected into a suitable target cell which will express the peptide. Transfection techniques known in the art include, but are not limited to, calcium phosphate precipitation (87) and electroporation (88). A variety of host-expression vector systems may be utilized to express the antibody proteins described herein preferably including eukaryotic cells.

The application discloses isolated nucleic acids encoding the antibodies disclosed or otherwise enabled herein. The nucleic acids may comprise DNA or RNA and may be wholly or partially synthetic or recombinant. Reference to a nucleotide sequence as set out herein encompasses a DNA molecule with the specified sequence, and encompasses a RNA molecule with the specified sequence in which U is substituted for T, unless context requires otherwise.

In another embodiment, the nucleic acid molecules which encode the antibodies disclosed in the present application also comprise nucleotide sequences that are, for example, at least 50% identical to the sequences disclosed herein. Also contemplated are embodiments in which a sequence is at least 60% identical, at least 70% identical, at least 75% identical, at least 80% identical, at least 85% identical, at least 90% identical, at least 91% identical, at least 92% identical, at least 93% identical, at least 94% identical, at least 95% identical, at least 96% identical, at least 97% identical, at least 98% identical, at least 99% identical, or at least 99.5% identical, to a sequence disclosed herein and/or which hybridize to a sequence disclosed herein under conditions of high or moderate stringency. The percent identity may be determined by visual inspection and mathematical calculation.

Stringency, including "high stringency," as used herein, includes conditions readily determined by the skilled artisan based on, for example, the length of the DNA. Generally, such conditions are defined as hybridization conditions of 50% formamide, 6xSSC at 42°C (or other similar hybridization solution, such as, e.g., Stark's solution, in 50% formamide at 42°C), and with washing at approximately 68°C with 0.2xSSC, 0.1% SDS. The skilled artisan will recognize that the temperature and wash solution salt concentration can be adjusted as necessary according to factors such as the length of the probe.

"Moderate stringency," as used herein, includes conditions that can be readily determined by those having ordinary skill in the art based on, for example, the length of the DNA. The basic conditions are set forth by Sambrook et al. (79) and include use of a prewashing solution for the nitrocellulose filters 5xSSC, 0.5% SDS, 1.0 mM EDTA (pH 8.0), hybridization conditions of 50% formamide, 6xSSC at 42°C (or other similar hybridization solution, such as Stark's solution, in 50% formamide at 42°C), and washing conditions of 60°C, 0.5xSSC, 0.1% SDS.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567).

Methods of making antibody fragments are also known in the art (89). Antibody fragments of the present invention can be prepared by proteolytic hydrolysis of the antibody or by expression in E. coli of DNA encoding the fragment. Antibody fragments, as noted above, can be obtained by pepsin or papain digestion of whole antibodies conventional methods. For example, antibody fragments can be produced by enzymatic cleavage of antibodies with pepsin to provide a 5S fragment denoted F(ab')₂. This fragment can be further cleaved using a thiol reducing agent, and optionally a blocking group for the sulfhydryl groups resulting from cleavage of disulfide linkages, to produce 3.5S Fab' monovalent fragments. Alternatively, an enzymatic cleavage using pepsin produces two monovalent Fab' fragments and an Fc fragment directly. These methods are described, for example, in U.S. Patent No. 4,036,945 and U.S. Patent No. 4,331,647, and references contained therein.

Other methods of cleaving antibodies, such as separation of heavy chains to form monovalent light-heavy chain fragments, further cleavage of fragments, or other enzymatic, chemical, or genetic techniques may also be used, so long as the fragments bind to the conformational epitope that is recognized by the intact antibody. For example, Fv fragments comprise an association of VH and VL chains. This association may be noncovalent or the variable chains can be linked by an intermolecular disulfide bond or cross-linked by chemicals such as glutaraldehyde. Preferably, the Fv fragments comprise VH and VL chains connected by a peptide linker. These single-chain antigen binding proteins (sFv) are prepared by constructing a structural gene comprising DNA sequences encoding the VH and VL domains connected by an oligonucleotide. The structural gene is inserted into an expression vector, which is subsequently introduced into a host cell such as E. coli. The recombinant host cells synthesize a single polypeptide chain with a linker peptide bridging the two V domains. Another form of an antibody fragment is a peptide coding for a single CDR. CDR peptides ("minimal recognition units") are often involved in antigen recognition and binding. CDR peptides can be obtained by cloning or constructing genes encoding the CDR of an antibody of interest. Such genes are prepared, for example, by using the polymerase chain reaction to synthesize the variable region from RNA of antibody-producing cells.

The antibodies disclosed herein comprise engineered antibodies including fully human and humanized forms of non-human (e.g., primate or murine) antibodies. Such humanized antibodies are chimeric immunoglobulins, immunoglobulin chains or fragments thereof (such as Fv, Fab, Fab', F(ab')₂ or other antigen-binding subsequences of antibodies) that contain minimal sequences derived from non-human immunoglobulin. For the most part, humanized antibodies are human immunoglobulins in which residues from CDRs of the human acceptor antibody are replaced by residues from the donor antibody CDRs of a nonhuman species such as mouse, rat or rabbit having the desired specificity, affinity and capacity. An example of a humanized antibody of the presently disclosed inventive concepts is a humanized antibody comprising the CDRs of the G4 antibody and comprising human framework sequences which are homologous to the framework sequences of the G4 antibody.

In making an engineered antibody, a DNA sequence encoding an antibody molecule of the presently disclosed inventive concepts is prepared synthetically by established standard methods. For example, according to the phosphoamidine method, oligonucleotides are synthesized, e.g. in an automatic DNA synthesizer, purified, annealed, ligated and cloned in suitable vectors.

The DNA sequence may then be inserted into a recombinant expression vector, which may be any vector, which may conveniently be subjected to recombinant DNA procedures. The choice of vector will often depend on the host cell into which it is to be introduced. Thus, the vector may be an autonomously replicating vector, i.e., a vector that exists as an extrachromosomal entity, the replication of which is independent of chromosomal replication, e.g., a plasmid. Alternatively, the vector may be one which, when introduced into a host cell, is integrated into the host cell genome and replicated together with the chromosome(s) into which it has been integrated.

In the vector, the DNA sequence encoding the protein should be operably connected to a suitable promoter sequence. The promoter may be any DNA sequence, which shows transcriptional activity in the host cell of choice and may be derived from genes encoding proteins either homologous or heterologous to the host cell. Examples of suitable promoters for directing the transcription of the coding DNA sequence in mammalian cells include, but are not limited to, the LTR promoter, SV 40 promoter, the MT-1 (metallothionein gene) promoter or the adenovirus 2 major late promoter. A suitable promoter for use in insect cells is the polyhedrin promoter. Suitable promoters for use in yeast host cells include promoters from yeast glycolytic genes or alcohol dehydrogenase genes or the TPI1 or ADH2-4c promoters. Suitable promoters for use in filamentous fungus host cells are, for instance, the ADH3 promoter or the tpiA promoter.

The DNA coding sequence may also be operably connected to a suitable terminator, such as the human growth hormone terminator or (for fungal hosts) the TPI1 or ADH3 promoters. The vector may further comprise elements such as polyadenylation signals (e.g. from SV 40 or title adenovirus 5 Elb region), transcriptional enhancer sequences (e.g. the SV 40 enhancer) and translational enhancer sequences (e.g., ones encoding adenovirus VA RNAs).

The recombinant expression vector may further comprise a DNA sequence enabling the vector to replicate in the host cell in question. An example of such a sequence (when the host cell is a mammalian cell) is the SV 40 origin of replication. The vector may also comprise a selectable marker, e.g. a gene the product of which complements a defect in the host cell, such as the gene coding for dihydrofolate reductase (DHFR) or one which confers resistance to a drug, e.g. neomycin, hydromycin or methotrexate.

The procedures used to ligate the DNA sequences coding the proteins, the promoter and the terminator, respectively, and to insert them into suitable vectors containing the information necessary for replication, are well known to persons skilled in the art.

To obtain recombinant proteins of the presently disclosed inventive concepts the coding DNA sequences may be usefully fused with a second peptide coding sequence and a protease cleavage site coding sequence, giving a DNA construct encoding the fusion protein, wherein the protease cleavage site coding sequence positioned between the HBP fragment and second peptide coding DNA, inserted into a recombinant expression vector, and expressed in recombinant host cells. In one embodiment, said second peptide selected from, but not limited by the group comprising glutathion-S-reductase, calf thymosin, bacterial thioredoxin or human ubiquitin natural or synthetic variants, or peptides thereof. In another embodiment, a peptide sequence comprising a protease cleavage site may be the Factor Xa, with the amino acid sequence IEGR, enterokinase, with the amino acid sequence DDDDK, thrombin, with the amino acid sequence LVPR/GS, or Acharombacter lyticus, with the amino acid sequence XKX, cleavage site.

The host cell into which the expression vector is introduced may be any cell which is capable of expression of the peptides or full-length proteins, and is preferably a eukaryotic cell, such as invertebrate (insect) cells or vertebrate cells, e.g. Xenopus laevis oocytes or mammalian cells, in particular insect and mammalian cells. Examples of suitable mammalian cell lines include, but are not limited to, the HEk293 (ATCC CRL-1573), COS (ATCC CRL-1650), BHK (ATCC CRL-1632, ATCC CCL-10) or CHO (ATCC CCL-61) cell lines. Methods of transfecting mammalian cells and expressing DNA sequences introduced in the cells are well known in the art.

Alternatively, fungal cells (including yeast cells) may be used as host cells. Examples of suitable yeast cells include cells of *Saccharomyces* spp. or *Schizosaccharomyces* spp., in particular strains of *Saccharomyces cerevisiae.* Examples of other fungal cells are cells of filamentous fungi, e.g. *Aspergillus* spp. or *Neurospora* spp., in particular strains of *Aspergillus oryzae* or *Aspergillus niger.* The use of *Aspergillus* spp. for the expression of proteins is described in, e.g., EP 238 023.

The medium used to culture the cells may be any conventional medium suitable for growing mammalian cells, such as a serum-containing or serum-free medium containing appropriate supplements, or a suitable medium for growing insect, yeast or fungal cells, or any cell used to express the proteins. Suitable media are available from commercial suppliers or may be prepared according to published recipes.

The proteins recombinantly produced by the cells may then be recovered from the culture medium by conventional procedures including separating the host cells from the medium by centrifugation or filtration, precipitating the proteinaceous components of the supernatant or filtrate by means of a salt, e.g. ammonium sulphate, purification by a variety of chromatographic procedures, e.g. HPLC, ion exchange chromatography, affinity chromatography, or the like.

Once the antibodies have been obtained, for example once individual B cells have been identified and/or monoclonal antibodies have been produced, the sequences encoding the variable regions of these antibodies can be obtained. The variable region sequences can for example be obtained by first sequencing the antibody protein produced by the hybridoma, B-cell or phage and determining the encoding nucleic acid sequence. In one embodiment, the immunoglobulin variable region (VH and VL) DNA or cDNA may be sequenced instead. Where the antibody is derived from a hybridoma cell line or isolated B-cell, the cDNAs encoding the variable regions may be amplified using PCR by for example the methods described in Babcook et al. (Proc. Natl. Acad. Sci. USA, 93:7843-7848 (1996)), and in PCT Publication No. WO 92/02551.

A "chimeric" antibody refers to an antibody made up of components from at least two different sources. In certain embodiments, a chimeric antibody comprises a portion of an antibody derived from a first species fused to another molecule, e.g., a portion of an antibody derived from a second species. In certain such embodiments, a chimeric antibody comprises a portion of an antibody derived from a non-human animal fused to a portion of an antibody derived from a human. In certain such embodiments, a chimeric antibody comprises all or a portion of a variable region of an antibody derived from one animal fused to a portion of an antibody from a second animal. For example but not by way of limitation, a chimeric antibody may comprise all or portion of a variable region of an antibody derived from a non-human animal fused to a constant region of an antibody derived from a human.

Utilization of the monoclonal antibodies of the presently disclosed inventive concepts may require administration thereof to a subject, such as but not limited to, a human. However, when the monoclonal antibodies are produced in a non-human animal, such as a rodent, administration of such antibodies to a human patient will normally elicit an immune response, wherein the immune response is directed towards the sequence of the antibodies. Such reactions limit the duration and effectiveness of such a therapy. In order to overcome such problem, the monoclonal antibodies of the presently disclosed inventive concepts are "humanized", that is, the antibodies are engineered such that one or more antigenic portions thereof are removed and like portions of a human antibody are substituted therefore, while the antibodies' affinity for the desired epitope is retained. This engineering may only involve a few amino acids, or may include entire framework regions of the antibody, leaving only the complementarity determining regions of the antibody intact. Several methods of humanizing antibodies are known in the art and are disclosed in US Patent Nos. 6,180,370, issued to Queen et al. on January 30, 2001; 6,054,927, issued to Brickell on April 25, 2000; 5,869,619, issued to Studnicka on February 9, 1999; 5,861,155, issued to Lin on January 19, 1999; 5,712,120, issued to Rodriquez et al. on January 27, 1998; and 4,816,567, issued to Cabilly et al. on March 28, 1989.

As mentioned above, a "humanized" antibody refers to a non-human antibody that has been modified so that it more closely matches (in amino acid sequence) a human antibody. As described above, antibodies interact with target antigens predominantly through amino acid residues that are located in the heavy and light chain complementarity determining regions (CDRs). For this reason, the amino acid sequences within CDRs may be more diverse between individual antibodies than sequences outside of CDRs. Because CDR sequences are responsible for most antibody-antigen interactions, it is possible to express recombinant antibodies that mimic the properties of specific, naturally occurring antibodies by constructing expression vectors in which the CDR sequences from the naturally occurring antibody are grafted into framework sequences from a different antibody with different properties, such as human antibody framework regions. Such framework sequences can be obtained from public DNA databases or published references that include germline antibody gene sequences. For example, germline DNA sequences for human heavy and light chain variable region genes can be found in the "VBase" human germline sequence database (available on the Internet at mrc-cpe.cam.ac.uk/vbase), as well as in Kabat, E. A., et al. (1991) Sequences of Proteins of Immunological Interest, Fifth Edition, U.S. Department of Health and Human Services, NIH Publication No. 91-3242; Tomlinson, I. M., et al. (1992) "The Repertoire of Human Germline VH Sequences Reveals about Fifty Groups of VH Segments with Different Hypervariable Loops" J. Mol. Biol. 227:776-798; and Cox, J. P. L. et al. (1994) "A Directory of Human Germ-line VH Segments Reveals a Strong Bias in their Usage" Eur. J. Immunol. 24:827-836.

Humanized forms of antibodies are immunoglobulins, immunoglobulin chains or fragments thereof (such as Fv, Fab, Fab', F(ab')₂, or other antigen-binding subsequences of antibodies) that are principally comprised of the sequence of a human immunoglobulin, and contain minimal sequence derived from a non-human immunoglobulin. Humanization can be performed following the method of Winter and co-workers (Jones et al., 1986; Riechmann et al., 1988; Verhoeyen et al., 1988), by substituting rodent CDR sequences for the corresponding sequences of a human antibody. (See also U.S. Patent No. 5,225,539.) In some instances, Fᵥ framework residues of the human immunoglobulin are replaced by corresponding non-human residues. Humanized antibodies can also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the framework regions are those of a human immunoglobulin consensus sequence. The humanized antibody optimally also will comprise at least a portion of an immunoglobulin constant region (Fc), typically that of a human immunoglobulin (Jones et al., 1986; Riechmann et al., 1988; and Presta, 1992).

The presently disclosed inventive concepts further include the use of fully human monoclonal antibodies. Fully human antibodies essentially relate to antibody molecules in which the entire sequence of both the light chain and the heavy chain, including the CDRs, arise from human genes. Such antibodies are termed "human antibodies" or "fully human antibodies" herein. "Human antibodies" contain human antibody sequences and do not contain antibody sequences from a non-human animal. In certain embodiments, a human antibody may further contain synthetic sequences not found in native antibodies. The term is not limited by the manner in which the antibodies are made.

Human monoclonal antibodies may be prepared by the trioma technique; the human B-cell hybridoma technique (see Kozbor et al., Hybridoma, 2:7 (1983)) and the EBV hybridoma technique to produce human monoclonal antibodies (see Cole et al., PNAS, 82:859 (1985)). Human monoclonal antibodies may be utilized in the practice of the presently disclosed and claimed inventive concept(s) and may be produced by using human hybridomas (see Cote et al. PNAS, 80:2026 (1983)) or by transforming human B-cells with Epstein Barr Virus in vitro (see Cole et al., 1985).

In addition, human antibodies can be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example but not by way of limitation, in U.S. Patent Nos. 5,545,807; 5,545,806; 5,569,825; 5,625,126; 5,633,425; 5,661,016, and in Marks et al., J Biol. Chem., 267:16007 (1992); Lonberg et al., Nature, 368:856 (1994); Morrison, 1994; Fishwild et al., Nature Biotechnol., 14:845 (1996); Neuberger, Nat. Biotechnol., 14:826 (1996); and Lonberg and Huszar, Int Rev Immunol., 13:65 (1995).

Human antibodies may additionally be produced using transgenic nonhuman animals which are modified so as to produce fully human antibodies rather than the animal's endogenous antibodies in response to challenge by an antigen. (See PCT Publication No. WO 94/02602). The endogenous genes encoding the heavy and light immunoglobulin chains in the nonhuman host have been incapacitated, and active loci encoding human heavy and light chain immunoglobulins are inserted into the host's genome. The human genes are incorporated, for example, using yeast artificial chromosomes containing the requisite human DNA segments. An animal which provides all the desired modifications is then obtained as progeny by crossbreeding intermediate transgenic animals containing fewer than the full complement of the modifications. One embodiment of such a nonhuman animal is a mouse, and is termed the XENOMOUSE™ as disclosed in PCT Publication Nos. WO 96/33735 and WO 96/34096. This animal produces B cells which secrete fully human immunoglobulins. The antibodies can be obtained directly from the animal after immunization with an immunogen of interest, as, for example, a preparation of a polyclonal antibody, or alternatively from immortalized B cells derived from the animal, such as hybridomas producing monoclonal antibodies. Additionally, the genes encoding the immunoglobulins with human variable regions can be recovered and expressed to obtain the antibodies directly, or can be further modified to obtain analogs of antibodies such as, for example, single chain Fv molecules.

An example of a method of producing a nonhuman host, exemplified as a mouse, lacking expression of an endogenous immunoglobulin heavy chain is disclosed in U.S. Patent No. 5,939,598, issued to Kucherlapati et al. on August 17, 1999. It can be obtained by a method including deleting the J segment genes from at least one endogenous heavy chain locus in an embryonic stem cell to prevent rearrangement of the locus and to prevent formation of a transcript of a rearranged immunoglobulin heavy chain locus, the deletion being effected by a targeting vector containing a gene encoding a selectable marker; and producing from the embryonic stem cell a transgenic mouse whose somatic and germ cells contain the gene encoding the selectable marker.

A method for producing an antibody of interest, such as a human antibody, is disclosed in U.S. Patent No. 5,916,771, issued to Hori et al. on June 29, 1999. It includes introducing an expression vector that contains a nucleotide sequence encoding a heavy chain into one mammalian host cell in culture, introducing an expression vector containing a nucleotide sequence encoding a light chain into another mammalian host cell, and fusing the two cells to form a hybrid cell. The hybrid cell expresses an antibody containing the heavy chain and the light chain.

The term "neutralizing antibody" or "antibody that neutralizes" refers to an antibody that reduces at least one activity of a polypeptide comprising the epitope to which the antibody specifically binds. In certain embodiments, a neutralizing antibody reduces an activity in vitro and/or in vivo. The term "antigen-binding site" refers to a portion of an antibody capable of specifically binding an antigen. In certain embodiments, an antigen-binding site is provided by one or more antibody variable regions.

The antibodies of the present invention preferably include one or more modifications which inactivate complement. The term "complement activity" broadly encompasses the biochemical and physiological activities associated with activation of the complement system, individual complement pathway associated molecules, as well as genes encoding these molecules. Therefore, complement activities include, e.g., structure and expression of a gene encoding a complement pathway molecule, biochemical activity (e.g., enzymatic or regulatory) of a complement pathway molecule, cellular activities that initiate or result from activation of the complement system, and presence of serum autoantibodies against complement pathway molecules. In the hSel001 antibody the preferred modification to inactivate complement is a replacement of a lysine residue with alanine at position 342 in the heavy chain constant region CH2. Other substitutions at the same position may include for example any of gly, leu, trp, tyr, pro, thr, ser, met, asp, asn, glu, gin, phe, ile, val, thr, and cys with the proviso that the substitution is also effective in eliminating the ability of the constant region to activate complement.

The terms "complement pathway associated molecules," "complement pathway molecules," and "complement pathway associated proteins" are used interchangeably and refer to the various molecules that play a role in complement activation and the downstream cellular activities mediated by, responsive to, or triggered by the activated complement system. They include initiators of complement pathways (i.e., molecules that directly or indirectly triggers the activation of complement system), molecules that are produced or play a role during complement activation (e.g., complement proteins/enzymes such as C3, C5, C5b-9, Factor B, MASP-1, and MASP-2), complement receptors or inhibitors (e.g., clusterin, vitronectin, CR1, or CD59), and molecules regulated or triggered by the activated complement system (e.g., membrane attack complex-inhibitory factor, MACIF). Thus, in addition to complement proteins noted above, complement pathway associated molecules also include, e.g., C3/C5 convertase regulators (RCA) such as complement receptor type 1 (also termed CR1 or CD35), complement receptor type 2 (also termed CR2 or CD21), membrane cofactor protein (MCP or CD46), and C4bBP; MAC regulators such as vitronectin, clusterin (also termed "SP40,40"), CRP, CD59, and homologous restriction factor (HRF); immunoglobulin chains such as Ig kappa, Ig lambda, or Ig gamma; C1 inhibitor; and other proteins such as CR3, CR4 (CD11b/18), and DAF (CD55).

### Dual Function Anti-P-selectin Antibodies

The presently disclosed inventive concepts are directed to "dual function" antibodies and fragments thereof which bind specifically to P-selectin and not only block the binding of PSGL-1 to P-selectin, but also dissociate preformed P-selectin/PSGL-1 complexes. The present disclosure describes a heretofore unrecognized antibody binding domain (a conformational epitope) within the lectin domain (e.g., carbohydrate recognition domain, CRD) of P-selectin to which the dual function antibodies (which may be chimeric, human or humanized antibodies, or fragments thereof for example) bind. The presently disclosed inventive concepts are also directed to anti-P-selectin antibodies which bind to the conformational epitope described herein and which have a dual function in (1) blocking binding of PSGL-1 to P-selectin and (2) causing dissociation of preformed P-selectin/PSGL-1 complexes. The presently disclosed inventive concepts in particular are directed to using such dual function anti-P-selectin antibodies or antibody fragments thereof in treatments of inflammatory, thrombotic or other conditions or disorders in primates (including humans) which involve platelet, sickled red cell, leukocyte, lymphocyte, and/or endothelial cell adhesion, wherein the condition or disorder comprises or is associated with (but not limited to) at least one of sickle cell vasoocclusive pain crisis, inflammatory bowel disease (e.g., Crohn's Disease, ulcerative colitis, and enteritis), arthritis (e.g., rheumatoid arthritis, osteoarthritis, and psoriatic arthritis), graft rejection, graft versus host disease, asthma, chronic obstructive pulmonary disease, psoriasis, dermatitis, sepsis, nephritis, lupus erythematosis, scleroderma, rhinitis, anaphylaxis, diabetes, multiple sclerosis, atherosclerosis, thrombosis, tumor metastasis, allergic reactions, thyroiditis, ischemic reperfusion injury (e.g., due to myocardial infarction, stroke, or organ transplantation), and conditions associated with extensive trauma, or chronic inflammation, such as for example, type IV delayed hypersensitivity, associated for example with infection by Tubercle bacilli, or systematic inflammatory response syndrome, or multiple organ failure.

Importantly, the use of such dual function antibodies in treating these inflammatory diseases will allow not only the prevention, reduction, or inhibition of inflammation, but will also provide a mechanism to treat ongoing inflammatory disease processes in that the antibodies can dissociate preformed P-selectin/PSGL-1. For example, in the case of sickle cell vasoocclusive pain crisis, the dual function antibody is able to not only prevent future vasoocclusive events, but can also be used in the treatment of ongoing vasoocclusion. The application also discloses screening assays for the detection of anti-P-selectin antibodies which bind to the conformational epitope of P-selectin newly described here, and which not only block binding of PSGL-1 to P-selectin but also cause reversal of the binding of PSGL-1 to P-selectin (i.e., dissociation of the preformed complex).

As noted herein, the presently disclosed inventive concepts are directed to purified antibodies (including but not limited to chimeric, human, or humanized antibodies and fragments thereof), which recognize (i.e., bind to) P-selectin (SEQ ID NO:1) and which block binding of P-selectin to PSGL-1 (or PSGL-1-like receptors) and further cause dissociation of preformed adhesions or cell complexes that were mediated through PSGL-1/P-selectin interactions, and therapeutic methods of using such antibodies (or binding fragments thereof)

More particularly, the presently disclosed inventive concepts are directed to purified antibodies (or fragments thereof), against P-selectin, host cells that produce such anti-P-selectin antibodies (or fragments thereof), screening assays to identify anti-P-selectin antibodies (or fragments thereof) which block leukocytes, sickled erythrocytes, lymphocyte, platelet and endothelial cell P-selectin-mediated adhesion and additionally cause dissociation of preformed adhesions or cell complexes that were mediated through PSGL-1/P-selectin interactions, and therapeutic methods using such antibodies (or binding fragments thereof). The presently disclosed inventive concepts include novel antibodies against primate (including human) P-selectin and binding fragments thereof, particularly including, but not limited to, G4, humanized forms of G4, and hSel001 antibodies. Preferred antibodies of the disclosure are capable of specifically binding primate (particularly human) P-selectin, and inhibiting one or more P-selectin activities in vitro and/or in vivo. Where used herein, the term "PSGL-1" is also intended to include "PSGL-1-like receptor" on sickled red cells (erythrocytes).

In general, an antibody or antibody fragment of the presently disclosed inventive concepts can have any upper size limit so long as it is has similar or immunological properties relative to antibody that binds with specificity to the P-selectin-binding site described herein and which blocks binding of PSGL-1 to P-selectin and dissociates preformed P-selectin/PSGL-1 complexes. Where used herein the term "inclusive" is intended to refer to all integers between any two numbers listed herein.

As noted elsewhere herein, antibody fragments of the presently disclosed inventive concepts retain the ability to selectively bind to all of or a portion of the P-selectin binding epitope described herein. Preferably, an antibody or binding fragment of an antibody of the present invention is capable of binding to an epitope comprising one or more of amino acid residues 1-35, or, more particularly, 4-23, of the sequence set forth in SEQ ID NO:1.

As noted above, the antibodies or antibody fragments of the presently disclosed inventive concepts in preferred embodiments comprise immunoglobulins of the isotypes IgG₁, IgG₂, IgG₃, IgG₄ or IgG₂/G₄ chimeras, preferably binds to P-selectin with a high affinity (for example wherein the K_{d} is ≤ 1000nM) and preferably comprises a human constant region, and preferably inhibits binding of P-selectin to PSGL-1 and more preferably also caused reversal of binding of P-selectin to PSGL-1 in a preformed complex. Further, the anti-P-selectin antibody or binding fragment thereof preferably does not activate complement via the classical pathway by interacting with C1q and preferably does not bind Fc receptors, collectively called antibody effector function. The presently disclosed inventive concepts in particular are directed to using such dual function anti-P-selectin antibodies or antibody fragments as described and identified herein in treatments for inflammatory, thrombotic or other conditions or disorders in primates (including humans) which involve platelet, sickled red cell, leukocyte, lymphocyte, and/or endothelial cell adhesion, wherein the condition or disorder comprises or is associated with (but not limited to) at least one of sickle cell vasoocclusive pain crisis, inflammatory bowel disease (e.g., Crohn's Disease, ulcerative colitis, and enteritis), arthritis (e.g., rheumatoid arthritis, osteoarthritis, and psoriatic arthritis), graft rejection, graft versus host disease, asthma, chronic obstructive pulmonary disease, psoriasis, dermatitis, sepsis, nephritis, lupus erythematosis, scleroderma, rhinitis, anaphylaxis, diabetes, multiple sclerosis, atherosclerosis, thrombosis, tumor metastasis, allergic reactions, thyroiditis, ischemic reperfusion injury (e.g., due to myocardial infarction, stroke, or organ transplantation), and conditions associated with extensive trauma, or chronic inflammation, such as for example, type IV delayed hypersensitivity, associated for example with infection by Tubercle bacilli, or systematic inflammatory response syndrome, or multiple organ failure.

As noted elsewhere herein, P-selectin plays a central role in recruitment of leukocytes and lymphocytes to inflammatory and thrombotic sites by binding to a surface ligand (PSGL-1) on these cells and in the binding of sickled red cells to endothelium having activated endothelial cells. PSGL-1 is constitutively expressed on leukocytes, including neutrophils and monocytes, and on some endothelial cells. A PSGL-1-like receptor is expressed on sickled red cells and enables these cells to bind P-selectin on activated endothelial cells.

Without wanting to be bound by theory, it is believed that the treatment of vasoocclusive sickle cell pain crisis, for example by the anti-P-selectin antibody of the present invention, is effective by inhibiting any one or more of the following interactions: (1) PSGL-1 on leukocytes binding to P-selectin on activated endothelium; (2) a PSGL-1-like ligand on sickled red cells binding to P-selectin on activated endothelium; (3) P-selectin on the surface of activated platelets binding PSGL-1 on endothelial cells; (4) sickled red cells binding leukocytes through an uncharacterized ligand-receptor interaction; (5) P-selectin on activated platelets binding the PSGL-1 like receptor on sickled red cells, and/or; (6) P-selectin on the surface of activated platelets binding PSGL-1 on leukocytes. It is believed that the dual function anti-P-selectin antibody blocks the initiation and propagation of vasoocclusion at multiple levels of cell-cell interactions in the microvasculature. Further, as noted elsewhere herein, the dual function anti-P-selectin antibody dissociates P-selectin/PSGL-1 complexes and thereby can be used therapeutically to intervene in ongoing vasoocclusion.

P-selectin mediates interactions of activated platelets or endothelial cells with blood cells including certain red blood cells (i.e. sickled red cells) and leukocytes including monocytes, neutrophils, eosinophils, CD4⁺T cells, CD8⁺T cells, B cells and natural killer (NK) cells. As noted herein, it is known that P-selectin is involved in a number of cellular responses to inflammation resulting from injury, infection, or physicochemical assaults. Atherosclerosis, characterized by atherosclerotic lesions on the inner surfaces of blood vessels, is one example of a condition involving the binding of certain leukocytes to P-selectin-bearing endothelial cells on the inner lining of blood vessel walls.

As indicated above, therapies directed to blocking P-selectin function, for example, using antibodies to P-selectin to prevent the tethering and rolling of leukocytes and adherence of red blood cells (i.e. sickled red cells), could have a profound effect on numerous types of inflammatory and thrombotic diseases. Given its pivotal roll in the initiation of rolling and tethering of leukocytes to the endothelium and platelets, P-selectin is a primary target for therapeutic development to treat inflammatory and thrombotic disorders. For example, the transient nature of the acute phase of sickle cell anemia coupled with the recurrent chronic effects of organ damage and associated complications and morbidity suggests that a therapeutic intervention that exhibits both blocking initial adhesion due to binding of P-selectin and PSGL-1, and inducing dissociation of prior, ongoing or pre-established adhesion, would have novel application to this and other inflammatory and thrombotic diseases. The application discloses a method of using a conformational antibody binding epitope of P-selectin to screen for and identify "dual function" antibodies to P-selectin which not only block P-selectin-PSGL-1 binding, but which also cause dissociation of preformed P-selectin/PSGL-1 complex (and thus cell-cell complex), and the use of such antibodies for therapeutic treatment of diseases, such as, but not limited to, inflammatory and thrombotic diseases.

As noted above, novel conformational binding epitopes of P-selectin have been discovered as described herein. The discovery of these conformational binding epitopes have further led to the discovery of dual function anti-P-selectin antibodies (thus the antibodies may be referred to herein as "dual function" antibodies) which bind with high specificity to the conformational epitope and which not only block the binding of P-selectin and PSGL-1, but which also induce the dissociation of preformed P-selectin/PSGL-1 complexes (i.e., induce the reversal of P-selectin-PSGL-1 binding) thereby causing the dissociation of cell complexes such as leukocyte/endothelial cell, leukocyte/platelet, lymphocyte/endothelial cell, lymphocyte/platelet, sickled red cell/endothelial cell or sickled red cell/platelet complexes.

The binding regions for some antibodies to P-selectin have been previously mapped using constructs of large functional domains encompassing the lectin, epidermal growth factor (EGF) and consensus repeat (CR) regions of the native protein P-selectin in mouse and human (90, 70, 91-95). These results indicated the primary binding areas for some function-blocking antibodies to P-selectin were in the lectin binding domain, a region that spans amino acid residues 1-120 of the native P-selectin protein, or in the EGF domain spanning amino acids 121-154.

### Examples are provided herein below.

The present disclosure describes the discovery of novel nonlinear (e.g., conformational) epitopes by using mouse/human chimeras containing the lectin, EGF, CR1 and CR2 domains of P-selectin that were probed with function-blocking test antibodies to P-selectin. Previous work has shown that, at a minimum, expression of the lectin and EGF domains are required for proper folding and conformation of P-selectin constructs (91). A comparison of the amino acid sequences of human and mouse P-selectin indicated that there is homology in the lectin domain with a specific number of amino acid residue differences between human and mouse. Herein, three-dimensional (3-D) homology was used to compare the human and mouse lectin, EGF, CR1 and CR2 domains of P-selectin (Fig. 1) to identify amino acid differences between human and mouse P-selectins (sequences and numbering according to the mature proteins).

The method used 3-D modeling of P-selectin to compare the positions of amino acid differences between human and mouse P-selectin on the exposed surface of the protein and to identify clusters of amino acid differences between human and mouse in the lectin and EGF domains which are located on the surface of the folded protein. This 3-D method represents clusters of amino acid differences which result from juxtaposition of discontinuous amino acids brought into proximity to one another by folding of the protein. For example, some amino acids will form conformational epitopes by virtue of being on the same surface, e.g. face, of helical structures. Homology comparison of such clusters allowed for selection of amino acid substitutions to test the effect of such changes on the binding of function-blocking (PSGL-1 blocking) antibodies to human P-selectin.

The method further involved mapping of conserved restriction sites in the open reading frames of the cDNA to identify a strategy for constructing chimeric proteins that span the lectin, EGF, CR1 and CR2 domains and would enable substitution of single or multiple amino acids at specific sites in the human or mouse P-selectin to identify those amino acids which optimize antibody binding to human P-selectin. Chimeras were constructed with known molecular cloning techniques, using human and mouse P-selectin N-terminal regions spanning the ATG through CR2 domain with a suitable vector such as pBluescript (pBS-hPsel and pBS-mPsel). The chimeras were inserted into another suitable vector such as pIG1 (pIG-hPsel and pIG-mPsel) where the constructs were fused to the Fc region of human IgG1 containing the hinge, CH2 and CH3 region. These constructs preserved structures that are consistent with the native conformation of P-selectin. Thus domains that were exposed on the surface of the native protein were also present on the chimera constructs and thus served as putative epitopes for binding of test antibodies. Such constructs could be transfected and transiently expressed using molecular and cell expression techniques known to persons having ordinary skill in the art.

Using this method, test antibodies, can be evaluated for binding to the human/mouse chimeras using a method such as, but not limited to, fluorescence-activated cell sorting (FACS) and surface plasmon resonance (BIACORE) methods known to persons having ordinary skill in the art. The effects of changes in amino acids in various positions in the chimera constructs by substitution of mouse amino acids, for example, into the human sequence, conversely, or human amino acids into the mouse sequence, for example, that abrogated or enabled binding of antibodies directed to human P-selectin were evaluated and thus enabled identification of particular amino acids for optimal binding.

### Characterization of Chimera Constructs

Amino acids of mouse P-selectin which have been substituted into the human P-selectin sequence are indicated in boldface in the chimeras described below. Amino acids of human P-selectin which have been substituted into the mouse sequence are indicated in italicized boldface. Substitution of glutamine for histidine is indicated as underlined boldface.

### Native Protein Constructs

SEQ ID NO:1
   Human P-selectin lectin, EGF, CR1, CR2 domains
SEQ ID NO:2
   Mouse P-selectin lectin, EGF, CR1, CR2 domains- Amino acid differences from human in boldface. **Human/Mouse Chimera Constructs**
SEQ ID NO:3
   *Chimera-1*
   (mouse substitutions in human cluster A - **N**₄**N**₁₄**V**₁₇**F**₁₈**R**₂₀**R**₂₁**H**₂₂**F**₂₃)
SEQ ID NO:4
   *Chimera-2*
   (human cluster A to I₃₅ - mouse thereafter)
SEQ ID NO:5
   *Chimera-3*
   (substitutions in human cluster A - to mouse **N**₄**N**₁₄**V**₁₇**F**₁₈)
SEQ ID NO:6
   *Chimera-4*
   (substitutions in human cluster A - to mouse **R**₂₀**R**₂₁**H**₂₂**F**₂₃)
SEQ ID NO:7
   *Chimera-5*
   (single amino acid change - human H₄ to mouse **N**₄)
SEQ ED NO:8
   *Chimera-5Q*
   (substitution of **Q** for H₄ in cluster A - removes putative glycosylation site)
SEQ ID NO:9
   *Chimera-6*
   (human sequence to EGF - S₁₂₁ - mouse EGF, CR1 and CR2)
SEQ ID NO:10
   *Chimera-7*
   (human sequence to G₁₇₇ - mouse thereafter) 241 F**AYD**SSC**K**F**E**C**QP**G**YRAR**G**SNTLH**CT**G**SG**Q**W**SE**P**L**P**T**C**EAIA**
SEQ ID NO:11
   *Chimera-7B*
   (human sequence to end of EGF - V₁₅₆ - mouse thereafter)
SEQ ID NO:12
   *Chimera-8*
   (single amino acid change - human **I**₁₄ to mouse **N**₁₄)
SEQ ID NO:13
   *Chimera-9*
   (single amino acid change - human K₁₇ to mouse **V**₁₇)
SEQ ID NO:14
   *Chimera-10*
   (single amino acid change - human Y₁₈ to mouse **F**₁₈)
SEQ ID NO:15
   *Chimera-11*
   (single amino acid change - human Q₂₀ to mouse **R**₂₀)
SEQ ID NO:16
   *Chimera-12*
   (single amino acid change - human N₂₁ to mouse **R**₂₁)
SEQ ID NO:17
   *Chimera-13*
   (single amino acid change - human R₂₂ to mouse **H**₂₂)
SEQ ID NO:18
   *Chimera-14*
   (single amino acid change - human Y₂₃ to mouse **F**₂₃)
SEQ ID NO:19
   *Chimera-15*
   (human sequence to cluster C2 - S₉₇ - mouse thereafter)
SEQ ID NO:20
   *Chimera-16*
   (substitution of human ***H***₄***I***₁₄***K***₁₇***N***₂₁***R***₂₂ into mouse Cluster A)
SEQ ID NO:21
   *Chimera-17*
   (human sequence to Cluster B to I₃₅ - mouse Cluster B to I₄₂ - human to CR1 to E₁₅₄ - mouse CR1, CR2)
SEQ ID NO:22
   *Chimera-17B*
   (human sequence to Cluster B to I₃₅ - mouse Cluster B to I₄₂ - human thereafter)
SEQ ID NO:23
   *Chimera-18*
   (human sequence with mouse cluster C (C1, C2, C3) and mouse CR1, CR2)
SEQ ID NO:24
   *Chimera-18B*
   (human sequence with mouse cluster C (C1, C2, C3)
SEQ ID NO:25
   *Chimera-19*
   (human sequence with mouse Cluster D and mouse CR1, CR2)
SEQ ID NO:26
   *Chimera-19B*
   (human sequence with mouse Cluster D)
SEQ ID NO:27
   *Chimera-20*
   (human sequence with mouse Cluster E and mouse CR1, CR2)
SEQ ID NO:28
   *Chimera-20B*
   (human sequence with mouse Cluster E)
SEQ ID NO:29
   *Chimera-21*
   (human sequence with mouse Cluster F)

### FACS analysis of anti-P-selectin antibodies to human/mouse chimeras

Antibody binding to human/mouse chimeras of P-selectin was analyzed using FACS analysis on a system such as a BD BIOSCIENCES FACS ARIA CELL SORTER to measure binding of anti-P-selectin antibodies to human/mouse chimeras which were coupled to beads coated with a goat anti-human Fc antibody. Such beads coated with chimeras were incubated with test anti-P-selectin antibodies that were then detected with anti-mouse Fc or isotype specific antibodies labeled with reporters, such as FITC, suitable for detection by the FACS system.

### One-step Surface Plasmon Resonance (BIACORE)

In one aspect of the presently disclosed inventive concepts, BIACORE chips were used to capture a test anti-P-selectin antibody. Human-mouse P-selectin chimeras described herein were disposed onto the chip and test antibodies were added to the prebound chip. Binding of the chimeras to test antibodies was measured by resonance response units.

### Two-Step Surface Plasmon Resonance (BIACORE) Analysis

In another aspect of the presently disclosed inventive concepts, a capture chip, such as a BIACORE chip was provided with a goat anti-human IgG Fc polyclonal antibody covalently attached to its surface. P-selectin chimeric human/mouse constructs of the lectin, EGF, CR1 and CR2 domain on a human IgG Fc were injected onto the chip and captured at concentrations that achieve a standardized level of surface coating as measured by the resonance response. The resonance response level achieved after loading each P-selectin chimera construct was designated as a new "secondary baseline" level. Test anti-P-selectin antibodies (e.g., mouse or humanized monoclonal anti-P-selectin antibodies, including G1, G3, G4, G5 and hSel001) were then injected onto the BIACORE chip and incubated for binding to the P-selectin chimera construct already captured on the surface. The method could be modified to test humanized antibodies by creating P-selectin constructs on mouse IgG Fc and capturing with a goat anti-mouse IgG Fc polyclonal antibody and then probing with test humanized anti-P-selectin antibodies. Antibodies which bind to the P-selectin constructs cause an increase of the resonance response level from the secondary baseline. The resulting increase in resonance response may be measured as "added resonance units (RUs)" and thus indicate the level of binding to the P-selectin construct pre-coated onto the capture chip of the test antibody. Using these methods, optimal requirements for the binding of anti-P-selectin antibodies to P-selectin chimeras were precisely mapped to particular conformational epitopes.

### Identification of dual function anti-P-selectin antibodies (antibodies that both block and dissociate binding of P-selectin to PSGL-1)

BIACORE analysis was also used to discover dual functionality of specific anti-P-selectin antibodies, i.e., as discussed above, they can both block and dissociate (reverse) binding interactions between P-selectin and PSGL-1. In this method, PSGL-1, or small molecule mimetics of the binding epitope of PSGL-1 such as a biotinylated glycosulfopeptide mimetic (e.g., GSP-6), or chimeric proteins containing the N-terminus of PSGL-1, are captured on a BIACORE chip, such as a streptavidin chip, using methods known to persons having ordinary skill in the art (GSP-6 is a glycosylated, sulfated 18 amino acid peptide mimetic of amino acids 42-60 of the exposed N-terminus of PSGL-1 described in detail in U.S. Patent No. 6,593,459, for example). First, to demonstrate "function-blocking" ability, in one embodiment, an anti-P-selectin antibody is pre-mixed with soluble P-selectin and incubated for a period to allow formation of the P-selectin/antibody complex. The resulting anti-P-selectin antibody/P-selectin complex is introduced onto the chip or other substrate bearing the PSGL-1 (or PSGL-1 mimetic) and binding to the PSGL-1 or its mimetic is measured. Anti-P-selectin antibodies, which prevent binding of P-selectin to the PSGL-1 or PSGL-1 mimetic on the chip, are designated herein as function-blocking antibodies.

Second, anti-P-selectin antibodies which have been shown (by the above-method or another similar method) to be function-blocking antibodies (i.e., which block PSGL-1 binding to P-selectin), can be tested for an additional function, that is, having the ability to dissociate (reverse) binding between preformed P-selectin PSGL-1 complex. Such antibodies can be tested for "dual function" properties using the method of BIACORE analysis discussed herein. In one embodiment, to demonstrate the dual function property, PSGL-1, or a mimetic thereof such as GSP-6, is coupled to a BIACORE chip. P-selectin is then disposed on the chip and allowed to bind to the PSGL-1, or the mimetic. After equilibrium binding of P-selectin to PSGL-1, or the mimetic, is indicated by the sensogram, function-blocking anti-P-selectin antibodies are introduced and the dissociation of P-selectin binding to PSGL-1, or the mimetic is measured by any appropriate method. Such antibodies that are shown to dissociate (i.e., reverse), P-selectin/PSGL-1 binding are designated as "dissociating antibodies" and are characterized as dual function antibodies, i.e., they possess both function-blocking and dissociating properties in disrupting binding of P-selectin to PSGL-1. Such dual function antibodies are a particularly preferred embodiment of the invention as they are especially suitable for therapeutic application as treatments of acute and chronic inflammatory and thrombotic diseases such as are described elsewhere herein.

### Discovery of Conformational Epitopes

The three-dimensional (3-D) structure of the mature human and mouse P-selectin proteins were analyzed and compared as to amino acid differences in the lectin and EGF domains. Six clusters of conformational amino acid differences were identified on exposed surfaces of the proteins. These were designated as clusters A, B, C, D, E and F (Fig. 1). The N-termini of human and mouse P-selectins spanning residues 1-35 contain 8 amino acid differences. Cluster A was arbitrarily defined by the boundary of the first amino acid difference (H₄) and the last amino acid difference (Y₂₃). Cluster A contains 20 amino acids and forms a rigid alpha helix with a cysteine bond near the N-terminus of the protein (see region "1" in Fig. 5). Cluster B (Fig. 1) is a conformational epitope spanning amino acid residues 36-42 and contains 4 amino acid differences between human and mouse P-selectin. Where used herein, the term "conformational epitope" is intended to refer to an epitope which is not recognized under reducing conditions. Clusters C and E (Fig. 1) are conformational and discontinuous and are brought into proximity by folding of the native P-selectin protein. Cluster C has three conformational regions (C1, C2, C3) containing 5 amino acid differences between human and mouse P-selectin. Cluster C1 is separated from C2 by 51 amino acids and cluster C2 is separated from C3 by 15 amino acids. Likewise cluster E has two conformational epitopes (E1, E2) containing five amino acid differences between human and mouse P-selectin with cluster E1 being separated from E2 by 19 amino acids. Clusters A, B, C, D and E lie within the consensus lectin domain of P-selectin (Fig. 1). Cluster F resides in the EGF domain and has 3 amino acid differences out of 11 amino acids. Clusters C1, E1, C2, E2 and C3 encompass key contact residues which have previously been identified for interaction of P-selectin with its physiological ligand PSGL-1 (Somers et al). Clusters A and B are distal to (upstream of) these contact residues.

The open reading frames of cDNAs for human and mouse P-selectin were analyzed to identify common restriction sites that could be used to assemble chimeras spanning the clusters. PCR and chemical DNA synthesis was used to generate cDNAs coding for specific protein or chimera constructs such as SEQ ID NO: 1-29 (described above, and in the Sequence Listing). Restriction cloning was used to construct plasmids coding for the human/mouse chimeras. The chimeras were transiently expressed in COS-7 cells and utilized for FACS and BIACORE analysis. P-selectin chimeras were tested for binding function using BIACORE by analyzing their binding to GSP-6 bound to a BIACORE chip. As noted above, GSP-6 is a small molecule that mimics the N-terminus of human PSGL-1. All tested chimeras bound to the GSP-6 on the chip, though to varying degrees, as mouse P-selectin has a lower binding affinity to human PSGL-1 than does human P-selectin. This indicated that chimeras had maintained function after expression and purification.

### FACS Analysis

The results of FACS analysis of anti-P-selectin antibodies, using the constructs or chimeras corresponding to SEQ ID NOs.:1-29 are summarized in Table 1. Four anti-P-selectin test antibodies (G1, G3, G4 and G5) were isolated from hybridomas generated by immunization of mice with a human recombinant P-selectin containing the lectin and EGF domains (90, and unpublished data). These studies had shown that these antibodies were specific to human P-selectin and that G1, G3 and G4 are function-blocking antibodies while G5 is non-blocking (90, and unpublished data). G1, G3 and G5 were analyzed by FACS analysis. Using this method, G1, G3 and G5 all bound to human P-selectin (SEQ ID NO:1) but not to mouse P-selectin (SEQ ID NO:2). When the corresponding eight mouse amino acids were substituted in cluster A of the human sequence (Chimera 1, SEQ ID NO:3), binding by G1 but not G3 or G5 was abolished, indicating that at least one or more of the corresponding eight amino acid positions in cluster A was essential for binding of G1 to P-selectin and that the substitution with the "mouse" amino acids in those one or more positions abolished the binding. To confirm the importance of these residues, the eight different human amino acids in positions 1-23 were substituted in cluster A of the mouse sequence (SEQ ID NO:4, chimera-2). These substitutions conferred G1 binding to the mouse protein. Chimeras containing the human P-selectin lectin domain with mouse amino acid substitutions in the EGF, CR1 and CR2 domains (SEQ ID NO:9, chimera-6), and human sequence through the EGF domain and into the CR1 domain with mouse sequence in the remainder of CR1 domain and the entire CR2 domain (SEQ ID NO:10, chimera-7), were bound by G1 and G3 indicating that the primary binding epitopes remained intact after substitution of these mouse amino acids and that the conformation of the antibody binding epitopes were not adversely affected. These data confirmed that the binding epitopes for G1 and G3 resided in the lectin domain.

Using the FACS method, the test antibody G3 was shown to bind human P-selectin but not mouse P-selectin and in contrast to G1, bound to SEQ ID NO:3 (chimera-1), indicating that G3 binds to an epitope distinct from the epitope bound by G1. Specifics of the G3 epitope mapping are outlined in Table 1 below.

The test antibody G5, previously shown to be non-blocking, was also analyzed using this method. G5 was also shown to bind human P-selectin but not mouse P-selectin. G5 bound to SEQ ID NO:3 and SEQ ID NO:10 that spans the EGF domain and includes the first part of CR1 to N₁₇₈, but G5 did not bind to SEQ ID NO:9 that spans to S₁₂₈. These results indicate that antibody G5 binds to the first part of CR1 and requires at least amino acids R₁₂₈ through N₁₇₈.

**Table 1. Results of binding of various antibodies (G1, G3, G4, G5, hSel001) to human and mouse P-selectin and chimera constructs thereof. ¹By FACS, ²By BIACORE 1-step, ³By BIACORE 2-step, ^{w}Weak binding**

| Human/Mouse Chimeras | | | | | | | | |
|---|---|---|---|---|---|---|---|---|
| SeqID | Mouse Domains Inserted in Human P-selectin | Human | Mouse | G1 | G3 | G5 | G4 | hSel001 |
| 1 | None | 1-279 | 0 | +^{1,2,3} | +^{1,2,3} | +^{1,3} | +³ | +² |
| 2 | all | 0 | 1-282 | -^{1,2,3} | -^{1,2,3} | -^{1,3} | -³ | -² |
| 3 | A_{4,14,17,18,20, 21,22,23} | 1-3, 24-279 | 4-23 | -^{1,2,3} | +^{1,3} | +¹ | -³ | |
| 4 | B,C,D,E,F | 1-35 | 36-282 | +^{1,2,3} | -³ | | +³ | |
| 5 | A_{4,14,17,18} | 19-279 | 1-18 | -² | | | | |
| 6 | A_{20,23} | 1-19, 24-279 | 20-23 | -² | | | | |
| 7 | A₄ | 1-3, 5-279 | 4 | +^{2w,3w} | +³ | | +^{3w} | |
| 8 | none | 1-3, 5-279 | 0 | +^{2,3} | +³ | | +³ | |
| 9 | F, CR1, CR2 | 1-128 | 129-282 | +^{1,2,3} | +^{1,3} | -^{1,3} | +³ | |
| 10 | CR1, CR2 | 1-177 | 178-282 | +^{1,2,3} | +^{1,3} | +^{1,3} | +³ | |
| 11 | CR1, CR2 | 1-156 | 157-282 | +² | +² | -² | | |
| 12 | A₁₄ | 1-13, 15-279 | 14 | -² | | | | |
| 13 | A₁₇ | 1-16, 18-279 | 17 | -² | | | | |
| 14 | A₁₈ | 1-17, 19-279 | 18 | +² | | | | |
| 15 | A₂₀ | 1-19, 21-279 | 20 | +² | | | | |
| 16 | A₂₁ | 1-20, 22-279 | 21 | +^{2w} | | | | |
| 17 | A₂₂ | 1-21, 23-279 | 22 | -² | | | | |
| 18 | A₂₃ | 1-22, 24-270 | 23 | +² | | | | |
| 19 | C2,E2,C3, F,CR1,CR2 | 1-97 | 98-282 | +^{2,3} | -³ | | +³ | |
| 20 | B,C,D,E,F | 1-35 H/M hybrid | 36-282 | +^{2,3} | -^{2,3} | | +³ | +² |
| 21 | B,CR1,CR2 | 1-35, 43-156 | 36-42, 157-282 | +³ | +³ | -³ | | |
| 22 | B | 1-35, 43-279 | 36-42 | | +³ | +³ | | |
| 23 | C1,C2,C3,C R1,CR2 | 1-43, 47-97, 99-113, 115-156 | 44-46, 98, 114, 157-282 | +³ | -³ | -³ | | |
| 24 | C1,C2,C3 | 1-43, 47-97, 99-113, 115-279 | 44-46, 98, 114 | | -³ | +³ | | |
| 25 | D,CR1,CR2 | 1-55, 72-156 | 56-71, 157-282 | +³ | +³ | | | |
| 26 | D | 1-55, 72-279 | 56-71 | | +³ | +³ | | |
| 27 | E1,E2,CR1, CR2 | 1-84, 89-107, 113-156 | 85-88, 108-112, 157-282 | +³ | +³ | | | |
| 28 | E1,E2 | 1-84, 89-107, 113-279 | 85-88, 108-112 | | +³ | +³ | | |
| 29 | F | 1-128, 140-279 | 129-139 | | +³ | +³ | | |
| | | | Critical Amino Acid Positions: | A (4,14,17, 21,22) | c | First part of CR1 (157-164) | A (4,14,1 7,21,2 2) | A (4,14,17, 21,22) |

### One-step Surface Plasmon Resonance (BIACORE)

To further investigate the importance of the cluster A domain (amino acids 4-23) to the binding of the G1 antibody to P-selectin, several chimeric constructs were made in which single or multiple mouse amino acids were inserted into the human P-selectin sequence and binding was analyzed using the surface plasmon resonance ("one-step" BIACORE) methods disclosed herein. The one-step BIACORE binding results are presented in Table 1. The G1 test antibody was captured on a BIACORE chip and the binding of various chimeras was measured as response units. A representative sensogram showing binding of G1 to constructs of human P-selectin (SEQ ID NO:1), mouse P-selectin with human cluster A (SEQ ID NO:4) and mouse P-selectin (SEQ ID NO:2) is shown in Figure 2. Using this method, it was shown that G1 bound to human P-selectin and to SEQ ID NO:4 (where human amino acids were substituted in cluster A of mouse P-selectin), but did not bind to mouse P-selectin (SEQ ID NO:2). These results demonstrated that this method is consistent with the FACS results.

Mouse P-selectin (SEQ ID NO:2) has a putative glycosylation site (N) at position 4 whereas human P-selectin (SEQ ID NO:1) does not. To test the importance of this difference, position 4 of human P-selectin SEQ ID NO:1 was substituted with an N (forming SEQ ID NO:7) or a Q (forming SEQ ID NO:8) were and the effect of these substitutions on G1 antibody binding was measured. Inserting asparagine (N) into human P-selectin at position 4 diminished G1 binding suggesting that glycosylation at this site in human P-selectin would interfere with antibody binding. Substitution of glutamine (Q) at this position did not prevent G1 binding.

To further identify amino positions in cluster A that are optimal or essential for G1 antibody binding, single amino acid substitutions of mouse sequence amino acids into the human P-selectin (SEQ ID NO:1) were made, and binding of the resulting chimeras to G1 was measured using the one-step BIACORE method disclosed herein (Table 1). The chimeras tested (with specific substitutions indicated in parentheses) were: SEQ ID NO:7 (H₄N); SEQ ID NO:12 (I₁₄N); SEQ ID NO:13 (K₁₇V); SEQ ID NO:14 (Y₁₈F); SEQ ID NO:15 (Q₂₀R); SEQ ID NO:16 (N₂₁R); SEQ ID NO:17 (R₂₂H); and SEQ ID NO:18 (Y₂₃F). The G1 antibody bound to SEQ ID NO:14, 15 and 18, but did not bind SEQ ID NO:12, 13, and 17 and only weakly to SEQ ID NO:7 and SEQ ID NO:16. This result indicated that amino acid positions 4, 14, 17, 21, and 22 are each individually required positions for G1 binding. In a preferred embodiment, these amino acids are H₄, I₁₄, K₁₇, N₂₁ and R₂₂, respectively.

To confirm that the humanized form of G1 (hSel001) maintained the identical epitope specificity of the parental antibody, the binding of hSel001 to SeqID: 1, 2 and 20 was tested using this method. The binding pattern of hSel001 was identical to that of G1 confirming that the epitope specificity was maintained during the humanization process.

### Two-step Surface Plasmon Resonance (BIACORE)

To assess G1, G3, G4 and G5 (G5 is non-blocking) binding to additional chimeras, the two-step surface plasmon resonance ("two-step" BIACORE) assay described herein was used. The results of the "two-step" assays for the test antibodies are presented in Table 1, and in Fig. 2. Using this method, none of the test antibodies investigated bound to mouse P-selectin and all bound to human P-selectin demonstrating their specificity to human P-selectin. G1, G3, G4 and G5 test antibodies all bound to SEQ ID NO:10 indicating they all bind to a region spanning the N-terminus through the lectin and EGF domains of human P-selectin. The G5 non-blocking antibody did not bind to SEQ ID NO:9, but did bind SEQ ID NO:10 confirming that G5 binds to the CR1 domain.

Further analysis using this method showed that G3 did not bind SEQ ID NO:23, which has mouse amino acids inserted in cluster C1, C2, C3, CR1, and CR2, nor did it bind SEQ ID NO:24 which has mouse amino acids in C1, C2, and C3. G3 also did not bind to other chimeras that had mouse sequence in cluster C, that is SEQ ID NO:19 and SEQ ID NO:20. These results indicate that the blocking G3 antibody requires cluster C for binding and demonstrates the novel finding that conformational clusters of amino acids brought into proximity by protein folding can serve as binding domains (conformational epitopes) for anti-P-selectin antibodies. The method also confirmed that G3 can block binding of PSGL-1 and P-selectin and thus has function-blocking properties (Fig. 3). However, the method also showed that G3 did not bind to or dissociate (reverse) the binding of P-selectin/PSGL-1 complex (Fig. 4) and thus does not have the dual function properties of the preferred antibodies of the presently disclosed inventive concepts.

Amino acid positions which contribute to the binding of G1 and G4 to P-selectin were identified by generating a human/mouse hybrid in cluster A. The hybrid cluster A chimera (SEQ ID NO:20) contains human P-selectin amino acids at positions 4, 14, 17, 21 and 22 (H, I, K, N, and R, respectively) and mouse P-selectin amino acids at positions 18, 20, and 23 (Y, Q, and Y, respectively). As indicated in Table 1, both G1 and G4 bound SEQ ID NO:20. This result when taken with the previous data indicates that amino acid positions 4, 14, 17, 21, and 22 comprise positions which are each required for optimal binding of G1, G4 and the humanized form of G1 (hSel001) to P-selectin. These results comprise a novel finding that a group of antibodies including G1, G4 and hSel001 bind the same or similar epitope and that this epitope is found in the helix structure of cluster A that is distal to the lectin-ligand binding domain contact residues previously identified for P-selectin (71). In a preferred embodiment the amino acids at positions 4, 14, 17, 21, and 22 are H, I, K, N, and R, respectively. 3-D analysis of this epitope revealed a rigid helical structure with the required amino acids occupying sites on the same face of the helix; thus cluster A is designated as comprising a conformational epitope (Fig. 5). BIACORE analysis shown in Figs. 3 and 4 confirmed that G1, G4 and hSel001 can block and also dissociate the binding of P-selectin and PSGL-1 and thus binding of the herewithin described epitope by this group of antibodies has the dual function properties of the preferred embodiments of the presently disclosed inventive concepts.

These results indicate that antibodies that bind to a conformational epitope located within amino acids 1-35, and more particularly within amino acids 4-23, of SEQ ID NO:1 which is distal to the lectin-ligand binding domain in human P-selectin, will have unique dual function properties. Without being be bound by theory, it is contemplated that the antibodies which bind to this epitope act by contributing allosteric forces that exert on the lectin-ligand binding interface to induce a conformational change that dissociates P-selectin binding to PSGL-1. Thus G1 is able to bind the distal epitope at cluster A and block and dissociate the complex by binding and disrupting the molecular interactions at the lectin-ligand binding site on P-selectin. In contrast, antibodies such as G3, that bind to an epitope in the lectin-ligand binding domain of P-selectin can block P-selectin binding to PSGL-1 by allosteric hindrance, but cannot cause dissociation of the P-selectin/PSGL-1 complex since the antibody cannot bind to the conformational epitope of Cluster C when it is occupied by the ligand. The test antibody G5 bound to the cluster of P-selectin CR1 and was shown to be non-blocking (Fig. 3).

In summary, antibodies which bind to P-selectin have been characterized as having three possible activities in regard to the interaction of P-selectin, and its ligand, PSGL-1.

First, P-selectin antibodies can bind to P-selectin but not interfere with the binding of PSGL-1 to P-selectin ("non-blocking"). For example, as shown herein, the antibody G5 binds amino acids 157-164 and requires R₁₅₇, E₁₆₁, L₁₆₂, E₁₆₃ and L₁₆₄ of CR1 for binding but this binding does not block P-selectin from binding to PSGL-1.

Second, P-selectin antibodies can bind to P-selectin and interfere with the binding of PSGL-1 to P-selectin ("function-blocking"), but not bind to or dissociate a preformed P-selectin/PSGL-1 complex. For example, the results described herein show that antibody G3 binds to conformational clusters in a different part of P-selectin that span the lectin-ligand binding domain. G3 binds cluster C and requires C1 amino acids Y₄₄, Y₄₅, S₄₆ and C2 amino acid P₉₈ and C3 amino acid H₁₁₄ and thus requires a conformational epitope. This antibody blocks the interaction between P-selectin and PSGL-1 but cannot bind to or dissociate preformed P-selectin/PSGL-1 complexes.

Third, we have discovered P-selectin antibodies with specific specificity that can bind to P-selectin and not only block the binding of PSGL-1 to P-selectin (function-blocking antibody) but can also cause reversal of preformed P-selectin/PSGL-1 binding (dissociative binding). Such antibodies are referred to herein as "dual function" antibodies. The results disclosed herein demonstrate, for example, that G1 binds a conformational epitope in cluster A, and that this binding had an absolute requirement for a conformational epitope comprising amino acid positions 4, 14, 17, 21, and 22, preferably wherein those amino acids are H, I, K, N, and R, respectively. As discussed elsewhere herein, substitution of the "human" amino acid (H, I, K, N, and R) at any one of these positions, respectively, with the corresponding "mouse" amino acid (N, N, V, R, and H) will result in the abrogation of binding by the dual function antibodies described herein.

In another embodiment of the presently disclosed inventive concepts, a previously uncharacterized mouse monoclonal anti-P-selectin antibody clone designated G4, generated using standard hybridoma methods, was tested for binding to the conformational epitope of cluster A, and was tested for dual function capabilities using the methods described herein. G4 was tested for binding to human/mouse chimeras SEQ ID NOs.:1-4, 7-10, 19 and 20. G4 was shown to bind SEQ ID NO:20 and had similar binding specificity as described for G1 (see Table 1). G4 was then shown to block binding of P-selectin to PSGL-1 (Fig. 3) and also to cause dissociation of preformed P-selectin/PSGL-1 complexes (Fig. 4), thus characterizing G4 as a dual function P-selectin antibody which binds an epitope (in cluster A) which is distal to the lectin-ligand binding domain of P-selectin and blocks and dissociates binding of P-selectin to PSGL-1. The G1 and G4 antibodies thus both bound to an epitope in cluster A and both demonstrated dual function properties. This result also demonstrates the use of cluster A or specific binding positions or amino acids thereof as an epitope able to be used to screen anti-P-selectin antibodies for dual function capabilities. Such dual function antibodies possess unique properties for therapeutic applications where initiation of P-selectin-mediated adhesion and/or ongoing P-selectin-mediated adhesion in acute or chronic settings may be treated. Using the methods described herein, other antibodies having dual function properties can be identified using the method of screening using the cluster A epitope or specific positions or amino acids thereof.

The humanized IgG2 anti-P-selectin antibody lacking effector function called hSel001 (a humanized P-selectin binding antibody comprising CDRs of mouse antibody G1 grafted into human framework regions as previously characterized in Publication No. WO 2008/069999) was also screened using the screening method described herein. A summary of the data (Table 1) shows that hSel001 antibody bound to the same chimeras (SEQ ID NO:1 and 20) as antibody G1. hSel001 binding was specific to the conformational epitope described herein located within cluster A. Results showed that antibody hSel001 possesses dual function properties enabling it to both block binding of P-selectin to PSGL-1 (Fig. 3) and dissociate preformed P-selectin/PSGL-1 complexes (Fig. 4). Thus hSel001 is another antibody optionally encompassed by the presently disclosed inventive concepts and can be used as a therapeutic treatment for inflammatory and thrombotic diseases as described herein, and wherein P-selectin binding to PSGL-1 is blocked, and dissociation of preformed P-selectin/PSGL-1 complex is promoted.

### Cell-based in vitro rolling assays under flow with human neutrophils

To further evaluate the blocking and dissociative properties of antibodies G1, G4 and hSel001, cell-based *in vitro* rolling assays were performed with freshly isolated human neutrophils that were introduced under a flow of 1.0 dyn/cm² in a flow chamber coated with low and high levels of membrane P-selectin. The low density P-selectin was coated at 0.25µg/ml and the high density P-selectin was coated at 2µg/ml. Site densities were determined using I¹²⁵-labeled G1 mAb to be 50 sites/mm² (low) and 380 sites/mm² (high). On low density P-selectin, neutrophils rolled at an average velocity of either 5µm/s or 6.5µm/s. On high density P-selectin, neutrophils rolled at an average velocity of 1µm/s. Neutrophils are introduced in buffer under flow and allowed to begin rolling and tethering. Once equilibrated, test antibodies (G1, G3, G4 and hSel001) were introduced in cell-free buffer under flow. There is a dead volume of about 1 minute interval before the antibody reaches the chamber. At 1 minute intervals thereafter, cells remaining bound are counted and expressed as % cells bound. Results were recorded on video microscopy for approximately 0-20 minutes and the data analyzed post run.

Results in Figure 6 panels (A) and (C) showed that neutrophils rolled at a higher velocity on low density P-selectin. Thus as the P-selectin/PSGL-1 complex released due to normal on/off kinetics of the lectin/ligand binding, neutrophils traveled greater distances at higher velocity to the next P-selectin binding site. As the complex releases, the previously occupied P-selectin becomes available for binding by anti-P-selectin antibodies. Thus all four antibodies, G1, G3, G4 and hSel001, showed equivalent blocking functionality over the course of 1-4 minutes.

Results in Figure 6 panels (B) and (D) on high density P-selectin showed that neutrophils roll much slower (1µm/s) as a greater number of P-selectin binding sites are available. Many neutrophils come to a rolling stop on P-selectin at this density. Under these conditions the murine antibodies G1 and G4 and the humanized antibody hSel001 were able to release rolling and tethering neutrophils by dissociating the P-selectin/PSGL-1 complex immediately and over the course of 1-8 minutes. In contrast the G3 anti-P-selectin antibody required up to 16-20 minutes to block rolling neutrophils. This indicated that the G3 antibody was only able to bind unoccupied P-selectin sites and thus block P-selectin/PSGL-1 complexes, but was not able to bind and dissociate the pre-formed complex. These cell-binding assays under flow confirm the BIACORE results reported previously herein which demonstrate that murine antibodies G1 and G4 and humanized antibody hSel001 all have dual function properties causing both blockage and dissociation of preformed P-selectin/PSGL-1 complexes.

### hSel001 has enhanced binding versus mouse antibody G1

The P-selectin binding affinities of G1 and hSel001 were analyzed in vitro and compared using surface plasmon resonance (Biacore). Soluble human P-selectin was covalently attached to a Biacore CM5 chip and the mouse antibody G1 and humanized antibody hSel001 were independently tested by introduction to the chip.

In order to further assess the interaction of the anti-P-selectin antibodies with P-selectin (antigen), kinetic analysis using surface plasmon resonance (SensiQ) was performed. The mouse monoclonal anti-P-selectin antibody G1 and the humanized hSel001 antibody were analyzed by surface plasmon resonance on SensiQ. To analyze the kinetics of binding of these two antibodies, the sensor chip was functionalized by covalently attaching recombinant protein G. The mouse antibody G1 was introduced and captured on the chip by injecting a 20nM solution for 1 minute. The humanized antibody hSel001 was introduced and captured on a similarly functionalized separate channel by injecting a 10nM solution for 1 minute. Concentrations of soluble human P-selectin (100nM-1.56nM) were introduced and binding measured as response units (RU). The data for binding of each was analyzed using Qdat analysis software.

Figure 7 shows binding results for both antibodies at a single P-selectin concentration.

Based on these results, the K_{d} (dissociation constant) for hSel001 was measured to be 5.89nM compared to a K_{d} of 8.94nM for the mouse antibody G1 (Table 2). This represents a 34% improvement in the binding affinity of the humanized anti-P-selectin antibody hSel001 when compared to the mouse monoclonal anti-P-selectin antibody G1. In addition the Kₐ (association constant) for hSel001 was higher by 64.7% than G1, meaning hSel001 binds more quickly to P-selectin. Both of these results indicate that the humanized hSel001 antibody has improved K_{d} (affinity) and Kₐ (speed of initial binding) versus G1.

| **Table 2-Kinetic Constants for P-Selectin** | | | | |
|---|---|---|---|---|
| **Antibody** | **ka (x10⁶ M⁻¹s⁻¹)** | **kd (x10⁻² s⁻¹)** | **KD (nM)** | **Res. SD (RU)** |
| G1 | 6.3±2 | 5.6±2 | 8.94±3 | 3.39 |
| **hSel001** | 2.22±1 | 1.306±6 | 5.89±2 | 4.48 |

Antibodies of the presently disclosed inventive concepts provided by any of the above described methods are preferably used in the manufacture of a pharmaceutical composition for use in the therapeutic treatment of a pathological condition, wherein such treatment comprises administering the pharmaceutical composition for mitigating, reversing, or inhibiting an inflammatory response or thrombosis or other condition.

It is an important objective of the presently disclosed inventive concepts to use the antibodies, or functionally active fragments or variants of said antibodies for the manufacture of a pharmaceutical composition and its use in the prevention and/or treatment of inflammatory responses or diseases or thrombosis such as, but not limited to, those described herein.

For example, the presently disclosed inventive concepts in particular are directed to, but not limited to, using such dual function anti-P-selectin antibodies or antibody fragments as described and identified herein in treatments for inflammatory, thrombotic or other conditions or disorders in primates (including humans) which involve platelet, sickled red cell, leukocyte, lymphocyte, and/or endothelial cell adhesion, wherein the condition or disorder comprises or is associated with (but not limited to) at least one of sickle cell vasoocclusive pain crisis, inflammatory bowel disease (e.g., Crohn's Disease, ulcerative colitis, enteritis), arthritis (e.g., rheumatoid arthritis, osteoarthritis, psoriatic arthritis), graft rejection, graft versus host disease, asthma, chronic obstructive pulmonary disease, psoriasis, dermatitis, sepsis, nephritis, lupus erythematosis, scleroderma, rhinitis, anaphylaxis, diabetes, multiple sclerosis, atherosclerosis, thrombosis, tumor metastasis, allergic reactions, thyroiditis, ischemic reperfusion injury (e.g., due to myocardial infarction, stroke, or organ transplantation), and conditions associated with extensive trauma, or chronic inflammation, such as for example, type IV delayed hypersensitivity, associated for example with infection by Tubercle bacilli, or systematic inflammatory response syndrome, or multiple organ failure. The term "primate" as used herein refers to humans, monkeys, including baboons and cynomolgus monkeys, and apes, the latter including chimpanzees, gorillas, gibbons and orangutans, for example. Other pathologic conditions not listed herein but which relate to inflammatory or thrombotic conditions or disclosures may also be treated using the antibodies and compositions described herein.

In the pharmaceutical composition of a medicament according to the presently disclosed inventive concepts, the antibodies may be formulated by any of the established methods of formulating pharmaceutical compositions, e.g. as described in the latest edition of Remington's Pharmaceutical Sciences or described elsewhere herein. The composition may typically be in a form suited for local or systemic injection or infusion and may, as such, be formulated with sterile water or an isotonic saline or glucose solution. The compositions may be sterilized by conventional sterilization techniques, which are well known in the art. The resulting aqueous solutions may be packaged for use or filtered under aseptic conditions and lyophilized, the lyophilized preparation being combined with the sterile aqueous solution prior to administration. The composition may contain pharmaceutically acceptable auxiliary substances as required to approximate physiological conditions, such as buffering agents, tonicity adjusting agents and the like, for instance sodium acetate, sodium lactate, sodium chloride, potassium chloride, calcium chloride, etc. The concentration of proteins may vary widely, for example, from less than about .01% to as much as 15-20% or more by weight. A unit dosage of the composition may contain for example from about 1 µg to about 1000 mg of an antibody or antibody fragment.

The antibodies or antibody fragments of the presently disclosed inventive concepts may be administered topically or by injection. Dosages will be prescribed by the physician according to the particular condition and the particular individual to be treated. Dosages and frequency is carefully adapted and adjusted according to parameters determined by the physician in charge. Preferred administration routes may be oral, via inhalation, subcutaneous, intravenous, intramuscular, intratracheal, intravesical, or intraperitoneal injections and may be given per 24 to 48 hours, or per week, every 14 days, every 4 weeks for example in the range of from 0.01-1000 mg, especially 0.1 mg to 100 mg, in particular 1-10 mg per kg body weight. The dose may be administered continuously through a catheter or in individual boluses. The antibody of the invention may be administered in an efficacious quantity such as, but not limited to, the ranges between 1 ng/kg to 1 µg/kg, 0.01 µg/kg to 50 µg/kg, 0.1 µg/kg to 1 µg/kg, 1 µg/kg to 5 µg/kg, 5 µg/kg to 10 µg/kg, 10 µg/kg to 50 µg/kg, 50 µg/kg to 100 µg/kg, 100 mg/kg to 1 mg/kg, 1 mg/kg to 10 mg/kg, or 10 mg/kg to 100 mg/kg body weight.

Pharmaceutical compositions used in the presently disclosed inventive concepts comprising antibodies described herein may additionally be supplemented by other therapeutic compounds which are routinely prescribed by the physician according to the particular condition and the particular individual to be treated such as an anti-inflammatory drug, wherein said drugs are prescribed by the physician according to the particular condition and the particular individual to be treated.

As noted elsewhere herein, the phenomenon of P-selectin/PSGL-1 binding has functional importance in sickled red cell, endothelial cell leukocyte and platelet interactions, and/or microvesicle adhesion, leukocyte rolling, recruitment, aggregation; leukocyte secretion of cytokines; promotion of coagulation; and other aspects of inflammation, thrombosis, coagulation, immune response, and signal transduction including , but not limited to, sickle cell vasoocclusive pain crisis, inflammatory bowel disease (e.g., Crohn's Disease, ulcerative colitis, enteritis), arthritis (e.g., rheumatoid arthritis, osteoarthritis, psoriatic arthritis), graft rejection, graft versus host disease, asthma, chronic obstructive pulmonary disease, psoriasis, dermatitis, sepsis, nephritis, lupus erythematosis, scleroderma, rhinitis, anaphylaxis, diabetes, multiple sclerosis, atherosclerosis, thrombosis, tumor metastasis, allergic reactions, thyroiditis, ischemic reperfusion injury (e.g., due to myocardial infarction, stroke, or organ transplantation), and conditions associated with extensive trauma, or chronic inflammation, such as for example, type IV delayed hypersensitivity, associated for example with infection by Tubercle bacilli, or systematic inflammatory response syndrome, or multiple organ failure. A neutralizing antibody (or fragment thereof) to P-selectin as described herein will inhibit one or more of these activities in a patient as mediated through P-selectin/PSGL-1 receptor binding (or in the case of sickled red cells, P-selectin/PSGL-1 like receptor binding), in vivo or in vitro, for example. Thus, the inhibition of P-selectin/PSGL-1 binding with a neutralizing antibody (or fragment thereof) described herein is useful in the treatment in a patient of various conditions and disorders including but not limited to, those described herein.

The P-selectin specific antibodies or binding fragments described herein can be linked to another molecule. For example, antibodies may be linked to another peptide or protein, toxin, radioisotope, cytotoxic or cytostatic agents. The antibodies can be linked covalently by chemical cross-linking or by recombinant methods. The antibodies may also be linked to one of a variety of nonproteinaceous polymers, e.g., polyethylene glycol, polypropylene glycol, or polyoxyalkylenes, in the manner set forth in U.S. Patent Nos. 4,640,835; 4,496,689; 4,301,144; 4,670,417; 4,791,192; or 4,179,337. The antibodies can be chemically modified by covalent conjugation to a polymer, for example, to increase their stability or half-life. Exemplary polymers and methods to attach them are also shown in U.S. Pat. Nos. 4,766,106; 4,179,337; 4,495,285; and 4,609,546.

The antibodies (or fragments thereof) may also be tagged with a detectable label. A detectable label is a molecule which, by its chemical nature, provides an analytically identifiable signal which allows the detection of a molecular interaction. A protein, including an antibody, has a detectable label if it is covalently or non-covalently bound to a molecule that can be detected directly (e.g., by means of a chromophore, fluorophore, or radioisotope) or indirectly (e.g., by means of catalyzing a reaction producing a colored, luminescent, or fluorescent product). Detectable labels include a radiolabel such as 1311 or 99Tc, a heavy metal, or a fluorescent substrate, such as Europium, for example, which may also be attached to antibodies using conventional chemistry. Detectable labels also include enzyme labels such as horseradish peroxidase or alkaline phosphatase. Detectable labels further include chemical moieties such as biotin, which may be detected via binding to a specific cognate detectable moiety, e.g., labeled avidin.

The application discloses methods of screening for anti-P-selectin antibodies and binding fragments thereof which block both P-selectin/PSGL-1 binding and/or which cause dissociation of preformed P-selectin/PSGL-1 complexes.

As noted above, the presently disclosed inventive concepts are directed to antibodies against P-selectin, host cells that produce such anti-P-selectin antibodies, vectors that contain DNA which encode such anti-P-selectin antibody expression and screening methods to identify anti-P-selectin antibodies which block P-selectin/PSGL-1 binding and in a further embodiment has a "dual function" in also causing dissociation of preformed P-selectin/PSGL-1 complex. Thus the application discloses methods of identifying anti-P-selectin antibodies that specifically bind to a conformational epitope in amino acids 1-35, and more preferably in amino acids 4-23, of human P-selectin (SEQ ID NO:1) (such as the conformational epitope described herein) and which block PSGL-1, or mimetics thereof, from binding to P-selectin, and which can reverse such binding thereto, thus exhibiting a dual function in blocking selectin-mediated adhesion due to P-selectin/PSGL-1 binding and in causing dissociation of preformed P-selectin/PSGL-1 complexes.

The screening method in preferred embodiments comprises *in vitro* fluid-based and/or substrate-based assays that can be used to identify anti-P-selectin antibodies or fragments thereof that inhibit or abolish P-selectin/PSGL-1 binding and preferably also cause dissociation of preformed P-selectin/PSGL-1 complexes. Test antibodies can be screened for dual function capability with a series of assays such as, but not limited to, those described herein which will identify those antibodies that bind to a conformational epitope within amino acids 1-35, and more particularly within amino acids 4-23, of P-selectin, and that block the binding of the PSGL-1 ligand to P-selectin, and which preferably cause dissociation of preformed P-selectin/PSGL-1 complexes. No anti-P-selectin antibodies have heretofore been shown to have the ability to both block PSGL-1 binding to P-selectin and to cause dissociation of preformed P-selectin/PSGL-1 complexes. Where used herein, the term "test antibody" refers to entire antibodies or fragments of antibodies. The test antibody can be a member of a library (e.g. phage, yeast, or bacteria) or an antibody fragment in a library. The library could be subtracted by eliminating all members that bound nondesired epitopes.

In a first step of the screening method, for example, test antibodies generated against P-selectin are assayed for the ability to block binding of PSGL-1 to P-selectin. Test antibodies which block binding of PSGL-1 to P-selectin are screened to determine their ability to cause dissociation of preformed P-selectin/PSGL-1 complexes. Test antibodies identified as having dual function of blocking both PSGL-1 binding to P-selectin, and causing dissociation of P-selectin/PSGL-1 complex comprise particularly preferred embodiments of the presently disclosed inventive concepts and can be used in the methods of the presently disclosed inventive concepts. Examples of fluid-based embodiments of the assays include, but are not limited to, (1) cell based FACS assays with leukocytes or HL60/activated platelets which measure cell aggregates which have or have not been exposed to an antibody able to bind to P-selectin (e.g., hSel001) or to a test antibody to demonstrate dissociation of the PSGL-1/P-selectin complex, (2) a liquid-based assay based on a P-selectin/GSP-6-streptavidin-biotin complex which has or has not been exposed to an antibody able to bind to P-selectin (e.g., hSel001) or to a test antibody, and measured with SEC (size exclusion chromatography), and (3) use of an AlphaLisa bead as a substrate but used in a liquid based assay.

In one embodiment of the method, test antibodies which block binding of PSGL-1 to P-selectin are first identified using a screening assay. For example, in a preferred embodiment of the screening assay, PSGL-1 or a synthetic PSGL-1 mimetic such as GSP-6, or a terminal epitope portion of PSGL-1 able to bind to P-selectin is provided (and optionally is bound to a support substrate such as a BIACORE chip), in a method known to persons having ordinary skill in the art. The PSGL-1 (or the PSGL-1 mimetic) (which may be bound to the substrate) is then exposed to an anti-P-selectin test antibody/P-selectin complex. The degree of binding of the complex to the PSGL-1-substrate is then evaluated. If the test antibody/P-selectin complex does not bind to the PSGL-1, the test antibody is identified as a "function-blocking" antibody. The GSP-6 or PSGL-1 mimetic, in one embodiment, is bound to biotin. The GSP-6/mimetic-biotin complex itself may be bound to a streptavidin coating on the substrate for example.

In another embodiment of the screening assay, P-selectin, or a portion thereof which maintains the integrity of the conformational epitope, is provided, and as above, is optionally bound to the support substrate. For example, a portion of P-selectin which maintains the conformational epitope includes, but is not limited to, the sequence comprising the lectin and EGF binding domains of P-selectin (e.g., amino acids 1-153 of SEQ ID NO:1). In this embodiment, the P-selectin or portion thereof with the conformational epitope is exposed to the test antibody, which binds to form the P-selectin-antibody complex. Then PSGL-1, or a high molecular weight mimetic thereof such as a GSP-6/biotin/avidin complex, is exposed to the P-selectin/test antibody complex and the degree of binding thereto of PSGL-1 (or the mimetic) is evaluated. An antibody which prevents or inhibits the binding of PSGL-1 to the P-selectin/antibody complex is identified as a "function-blocking" antibody.

In another embodiment of the screening assay, either PSGL-1 or a mimetic thereof is bound, as described above, to a support substrate (such as a bead or BIACORE chip). P-selectin is then applied to the PSGL-1/substrate to form the P-selectin/PSGL-1 (or mimetic) complex. The test antibody is then applied and dissociation of the complex is measured as a decrease in mass or as Response Units (RU) since P-selectin is being dissociated away. A function-blocking anti-P-selectin antibody, which induces dissociation of the P-selectin/PSGL-1 complex, is designated as a dual function anti-P-selectin antibody.

In an alternate embodiment of this screening assay, P-selectin or a portion of P-selectin comprising a conformational epitope may be bound to a support substrate rather than the PSGL-1. PSGL-1 or a high molecular weight mimetic thereof such as a GSP-6/biotin/avidin complex is then exposed to the P-selectin on the support substrate and allowed to form a PSGL-1/P-selectin complex. The PSGL-1/P-selectin complex is then exposed to a function-blocking anti-P-selectin antibody and dissociation of the complex is evaluated, for example using a BIACORE method as described elsewhere herein.

In yet another embodiment of the assay, the anti-P-selectin antibody itself is bound to the support substrate, and a P-selectin/PSGL-1 complex is exposed to it, and dissociation thereof is measured as above.

### References Cited

1. Springer TA. Traffic signals for lymphocyte recirculation and leukocyte emigration: the multistep paradigm. Cell, 1994. 76:301-314. PubMed: 7507411.
2. McEver RP, Moore KL, Cummings RD. Leukocyte trafficking mediated by selectin-carbohydrate interactions. J Biol Chem., 1995. 12;270(19): 11025-8. Review. PMID: 7538108.
3. Zimmerman GA, McIntyre TM, Prescott SM. Adhesion and signaling in vascular cell--cell interactions. J Clin Invest., 1996. 15;98(8): 1699-702. No abstract available. PMID: 8878418.
4. Frenette PS. Sickle cell vasoocclusion: heterotypic, multicellular aggregations driven by leukocyte adhesion. Microcirculation, 2004. 11(2):167-77. Review. PMID: 15280090.
5. Weyrich AS, Ma XY, Lefer DJ, Albertine KH, Lefer AM. In vivo neutralization of P-selectin protects feline heart and endothelium in myocardial ischemia and reperfusion injury. J Clin Invest., 1993. 91(6):2620-9. PMID: 7685773.
6. Lefer DJ. Pharmacology of selectin inhibitors in ischemia/reperfusion states. Annu Rev Pharmacol Toxicol., 2000. 40:283-94. Review. PMID: 10836137.
7. Singbartl K, Green SA, Ley K. Blocking P-selectin protects from ischemia/reperfusion-induced acute renal failure. FASEB J., 2000. 14(1):48-54. PMID: 10627279.
8. Palabrica T, Lobb R, Furie BC, Aronovitz M, Benjamin C, Hsu YM, Sajer SA, Furie B. Leukocyte accumulation promoting fibrin deposition is mediated in vivo by P-selectin on adherent platelets. Nature, 1992. 29;359(6398):848-51. PMID: 1279433.
9. Burger PC, Wagner DD. Platelet P-selectin facilitates atherosclerotic lesion development. Blood, 2003. 1;101(7):2661-6. Epub 2002 Dec 12. PMID: 12480714.
10. Théorêt JF, Bienvenu JG, Kumar A, Merhi Y. P-selectin antagonism with recombinant p-selectin glycoprotein ligand-1 (rPSGL-Ig) inhibits circulating activated platelet binding to neutrophils induced by damaged arterial surfaces. J Pharmacol Exp Ther., 2001. 298(2):658-64. PMID: 11454928.
11. Kumar A, Villani MP, Patel UK, Keith JC Jr, Schaub RG. Recombinant soluble form of PSGL-1 accelerates thrombolysis and prevents reocclusion in a porcine model. Circulation, 1999. 16;99(10):1363-9. PMID: 10077522.
12. Romano SJ. Selectin antagonists: therapeutic potential in asthma and COPD. Treat Respir Med., 2005. 4(2):85-94. Review. PMID: 15813660.
13. Grober JS, Bowen BL, Ebling H, Athey B, Thompson CB, Fox DA, Stoolman LM. Monocyte-endothelial adhesion in chronic rheumatoid arthritis. In situ detection of selectin and integrin-dependent interactions. J Clin Invest., 1993. 91(6):2609-19. PMID: 7685772.
14. Friedrich M, Bock D, Philipp S, Ludwig N, Sabat R, Wolk K, Schroeter-Maas S, Aydt E, Kang S, Dam TN, Zahlten R, Sterry W, Wolff G. Pan-selectin antagonism improves psoriasis manifestation in mice and man. Arch Dermatol Res., 2006. 297(8):345-51. Epub 2005 Dec 16. PMID: 16362415.
15. Johnson BA, Haines GK, Harlow LA, Koch AE. Adhesion molecule expression in human synovial tissue. Arthritis Rheum, 1993. 36(2):137-46. PMID: 7679271.
16. Bevilacqua MP, Nelson RM. Selectins. J Clin Invest., 1993. 91(2):379-87. Review. PMID: 7679406.
17. McEver RP, Beckstead JH, Moore KL, Marshall-Carlson L, Bainton DF. GMP-140, a platelet alpha-granule membrane protein, is also synthesized by vascular endothelial cells and is localized in Weibel-Palade bodies. J Clin Invest., 1989. 84(1):92-9. PMID: 2472431.
18. Sickle cell disease: screening, diagnosis, management, and counseling in newborns and infants. Clinical Practice Guideline No. 6. AHCPR Pub. No. 93-0562. Rockville, MD: Agency for Health Care Policy and Research, Public Health Service, U.S. Department of Health and Human
   Services. April 1993 [cited; Available from:
   www.ncbi.nlm.nih.gov/books/bv.fcgi?rid=hstat6.chapter.16946.
19. Nietert, PJ, Silverstein MD, Abboud MR. Sickle cell anaemia: epidemiology and cost of illness. Pharmacoeconomics, 2002. 20(6): p. 357-66. PMID: 12052095.
20. Bookchin, RM and Lew VL. Pathophysiology of sickle cell anemia. Hematol Oncol Clin North Am, 1996. 10(6): p. 1241-53. PMID: 8956013.
21. NHBLI. [cited; Available from:
   www.nhlbi.nih.gov/health/dci/Diseases/Sca/SCA_WhatIs.html.
22. NHLBI, The Management of Sickle Cell Disease. NIH Publication No.022117.
23. FDA Approves Droxia for Sickle Cell Anemia, in FDA Talk Paper. 1998. p. T98-11.
24. NIH, Hydroxurea Treatment for Sickle Cell Disease February 25-27 2008. National Institutes of Health Consensus Development Conference Statement, February 27 2008.
25. Gill FM, Sleeper LA, Weiner SJ, Brown AK, Bellevue R, Grover R, Pegelow CH, Vichinsky E. Clinical events in the first decade in a cohort of infants with sickle cell disease. Cooperative Study of Sickle Cell Disease. Blood, 1995. 86(2): p. 776-83. PMID: 7921116.
26. Ashley-Koch A, Yang Q, and Olney RS. Sickle hemoglobin (HbS) allele and sickle cell disease: a HuGE review. Am J Epidemiol, 2000. 151(9): p. 839-45. PMID: 10797557.
27. Thomas PW, Higgs DR, and Serjeant GR. Benign clinical course in homozygous sickle cell disease: a search for predictors. J Clin Epidemiol, 1997. Feb;50(2): p. 121-6. PMID: 9120504.
28. National Newborn Screening Status Report Updated 03/04/08.
29. Rice-Evans C, Omorphos SC, and Baysal E. Sickle cell membranes and oxidative damage. Biochem J, 1986. 237(1): p. 265-9. PMID: 3800879.
30. Wethers, D.L., Sickle cell disease in childhood: Part II. Diagnosis and treatment of major complications and recent advances in treatment. Am Fam Physician. 2000 Sep 15;62(6):1309-14. PMID: 11011859.
31. Gladwin MT, Sachev V, Jison ML, Shizukuda Y, Plehn JF, Minter K, Brown B, Coles WA, Nichols JS, Ernst I, Hunter LA, Blackwelder WC, Schechter AN, Rodgers GP, Castro O, Ognibene FP, Pulmonary hypertension as a risk factor for death in patients with sickle cell disease. N Engl J Med, 2004. 350(9): p. 886-95. PMID: 14985486.
32. Saborio P and Scheinman JI, Sickle cell nephropathy. J Am Soc Nephrol, 1999. 10(1): p. 187-92. PMID: 9890326.
33. Charache S, Eye disease in sickling disorders. Hematol Oncol Clin North Am, 1996. 10(6): p. 1357-62. PMID: 8956022.
34. Kaul, DK, Liu XD, Fabry ME, Nagel RL. Impaired nitric oxide-mediated vasodilation in transgenic sickle mouse. Am J Physiol Heart Circ Physiol, 2000. 278(6): p. H1799-806. PMID: 10843875.
35. Nolan, VG, Adewoye A, Baldwin C, Wang L, Ma Q, Wyszynski DF, Farrell JJ, Sebastiani P, Parrer LA, Steinberg MH. Sickle cell leg ulcers: associations with haemolysis and SNPs in Klotho, TEK and genes of the TGF-beta/BMP pathway. Br J Haematol, 2006. 133(5): p. 570-8. PMID: 1661647.
36. Nolan, VG, Wyszynski DF, Farrer LA, Steinberg MH. Hemolysis-associated priapism in sickle cell disease. Blood, 2005. 106(9): p. 3264-7. PMID: 15985542.
37. Gladwin MT and Kato GJ. Cardiopulmonary complications of sickle cell disease: role of nitric oxide and hemolytic anemia. Hematology Am Soc Hematol Educ Program, 2005: p. 51-7. PMID: 16304359.
38. Yale, SH, Nagib N, and Guthrie T. Approach to the vaso-occlusive crisis in adults with sickle cell disease. Am Fam Physician, 2000. 61(5): p. 1349-56, 1363-4. PMID: 10735342.
39. Heegaard, ED and Brown KE. Human parvovirus B19. Clin Microbiol Rev, 2002. 15(3): p. 485-505. PMID: 12097253.
40. Ballas, SK and Mohandas N. Pathophysiology of vaso-occlusion. Hematol Oncol Clin North Am, 1996. 10(6): p. 1221-39. PMID: 8956012.
41. Embury, SH. The not-so-simple process of sickle cell vasoocclusion. Microcirculation, 2004. 11(2): p. 101-13. PMID: 15280086.
42. Conran N and Costa FF. Hemoglobin disorders and endothelial cell interactions. Clin Biochem, 2009. 42(18): p. 1824-38. PMID: 19580799.
43. Chiang EY and Frenette PS. Sickle cell vaso-occlusion. Hematol Oncol Clin North Am, 2005. 19(5): p. 771-84, Review. PMID: 16214643.
44. Turhan A, Weiss LA, Mohandas N, Collier BS, Freette PS. Primary role for adherent leukocytes in sickle cell vascular occlusion: a new paradigm. Proc Natl Acad Sci U S A, 2002. 99(5): p. 3047-51. PMID: 1180644.
45. Hebbel RP, Osarogiagbon R, and Kaul D. The endothelial biology of sickle cell disease: inflammation and a chronic vasculopathy. Microcirculation, 2004. 11(2): p. 129-51. Review. PMID: 15280088.
46. Okpala I, Leukocyte adhesion and the pathophysiology of sickle cell disease. Curr Opin Hematol, 2006. 13(1): p. 40-4. Review. PMID: 16319686.
47. Matsui NM, Borsig L, Rosen SD, Yaghmai M, Varki A, Embury SH P-selectin mediates the adhesion of sickle erythrocytes to the endothelium. Blood, 2001. 98(6): p. 1955-62. PMID: 11535535.
48. Platt OS, Brambilla DJ, Rosse WF, Milner PF, Castro O, Steinberg MH, Klug PP. Mortality in sickle cell disease. Life expectancy and risk factors for early death. N Engl J Med, 1994. 330(23): p. 1639-44. PMID: 7993409.
49. Smith JA. Bone disorders in sickle cell disease. Hematol Oncol Clin North Am, 1996. 10(6): p. 1345-56. Review. PMID: 8956021.
50. Platt OS. Prevention and management of stroke in sickle cell anemia. Hematology Am Soc Hematol Educ Program, 2006: p. 54-7. Review. PMID: 17124040.
51. Maitre B, Habibi A, Roudot-Thoraval F, Bachir D, Belghiti DD, Galacteros F, Godeau B. Acute chest syndrome in adults with sickle cell disease. Chest, 2000. 117(5): p. 1386-92. PMID: 1087826.
52. Vichinsky EP, Neumayr LD, Earles AN, Williams R, Lennette ET, Dean D, Nickerson B, Orringer E, McKie V, Bellevue R, Daeschner C, Manci EA. Causes and outcomes of the acute chest syndrome in sickle cell disease. National Acute Chest Syndrome Study Group. N Engl J Med, 2000. 342(25): p. 1855-65. PMID: 10861320.
53. Serjeant GR, Ceulaer CD, Lethbridge R, Morris J, Singhal A, Thomas PW. The painful crisis of homozygous sickle cell disease: clinical features. Br J Haematol, 1994. 87(3): p. 586-91. PMID: 7993801.
54. Platt OS, Thorington BD, Brambilla DJ, Milner PF, Rosse WF, Vichinsky E, Kinney TR. Pain in sickle cell disease. Rates and risk factors. N Engl J Med, 1991. 325(1): p. 11-6. PMID: 1710777.
55. Health Advances, LLC. Application of Selexys Technologies to Inflammatory and Thrombotic Diseases. In Private Study. 2004.
56. McClish DK, Health related quality of life in sickle cell patients: the PiSCES project. Health Qual Life Outcomes, 2005. 3: p. 50. PMID: 16129027.
57. Charache S, Terrin ML, Moore RD, Dover GJ, Barton FB, Eckert SV, McMahon RP, Bonds DR. Effect of hydroxyurea on the frequency of painful crises in sickle cell anemia. Investigators of the Multicenter Study of Hydroxyurea in Sickle Cell Anemia. N Engl J Med, 1995. 332(20): p. 1317-22. PMID: 7715639.
58. Patient Information Leaflet: Droxia. Bristol-Myers Squibb Company, 2006.
59. NIH. NIH State-of-the-Science Conference Statement on Hydroxyurea Treatment for Sickle Cell Disease. NIH Consensus and State-of -the Science Statements; February 27-29, 2008. 25(1): 1-30. PMID: 18309362.
60. Hydroxyurea for the Treatment of Sickle Cell Disease, Agency for Healthcare Research and Quality; AHRQ Publication No. 08-E007. Feb. 2008.
61. Shet AS, Aras O, Gupta K, Hass MJ, Rausch DJ, Saba N, Koopmeiners L, Key NS, Hebbel RP. Sickle blood contains tissue factor-positive microparticles derived from endothelial cells and monocytes. Blood, 2003. 102(7): p. 2678-83. PMID: 12805058.
62. Solovey A, Gui L, Key NS, Hebbel RP. Tissue factor expression by endothelial cells in sickle cell anemia. J Clin Invest, 1998. 101(9): p. 1899-904. PMID: 9576754.
63. Solovey A, Lin Y, Browne P, Choong S, Wayner E, Hebbel RP. Circulating activated endothelial cells in sickle cell anemia. N Engl J Med, 1997. 337(22): p. 1584-90. PMID: 9371854.
64. Kaul DK and Hebbel RP. Hypoxia/reoxygenation causes inflammatory response in transgenic sickle mice but not in normal mice. J Clin Invest, 2000. 106(3): p. 411-20. PMID: 10930444.
65. Matsumoto S, Okabe Y, Setoyama H, Takayama K, Ohtsuka J, Funahashi H, Imaoka A, Okada Y, and Umesaki Y. Inflamatory bowel disease-like enteritis and caecitis in a senescence accelerated mouse P1/Yit strain. Gut, 1998. 43:71-78. PMID: 9771408.
66. Kosiewicz MM, Nast CC, Krishnan A, Rivera-Nieves J, Moskaluk CA, Matsumoto S, Kozaiwa K, and Cominelli F. Th1-type responses mediate spontaneous ileitis in a novel murine model of Crohn's disease. J. Clin. Invest., 2001 107:695-702. PMID: 11254669.
67. Rivera-Nieves J, Burcin TL, Olson TS, Morris MA, McDuffie M, Cominelli R and Ley K. Critical role of endothelial P-selectin glycoprotein ligand 1 in chronic murine ileitis. JEM, 2006. 203 (4): 907-917. PMID: 16567389.
68. Moore KL, Stults NL, Diaz S, Smith DF, Cummings RD, Varki A, McEver RP. Identification of a specific glycoprotein ligand for P- selectin (CD62) on myeloid cells. J Cell Biol, 1992;118:445-456. PMID: 1378449.
69. McEver RP, Cummings RD. Role of PSGL-1 binding to selectins in leukocyte recruitment. J Clin Invest, 1997;100:S97-103. PMID: 9413410.
70. Kansas GS. Selectins and their ligands: current concepts and controversies. Blood, 1996;88:3259-3287. PMID: 8896391.
71. Somers WS, Tang J, Shaw GD, Camphausen RT. Insights into the molecular basis of leukocyte tethering and rolling revealed by structures of P- and E-selectin bound to SLe(X) and PSGL-1. Cell, 2000. Oct 27;103(3):467-79. Erratum in: Cell, 2001. Jun 29;105(7):971. PMID: 11081633.
72. Mayadas TN, Johnson RC, Rayburn H, Hynes RO, Wagner DD. Leukocyte rolling and extravasation are severely compromised in P selectin-deficient mice. Cell, 1993. Aug 13;74(3):541-54. PMID: 7688665.
73. Xia L, Sperandio M, Yago T, McDaniel JM, Cummings RD, Pearson-White S, Ley K, McEver RP. P-selectin glycoprotein ligand-1-deficient mice have impaired leukocyte tethering to E-selectin under flow. J Clin Invest, 2002. 109:939-950. PMID: 11927621.
74. Yang J, Hirata T, Croce K, Merrill-Skoloff G, Tchernychev B, Williams E, Flaumenhaft R, Furie BC, Furie B. Targeted gene disruption demonstrates that P-selectin glycoprotein ligand 1 (PSGL-1) is required for P-selectin-mediated but not E-selectin-mediated neutrophil rolling and migration. J Exp Med, 1999. Dec 20;190 (12):1769-82. PMID: 10601352
75. Walcheck B, Moore KL, McEver RP, and Kishimoto TK. Neutrophil-neutrophil interactions under hydrodynamic shear stress involve L-selectin and PSGL-1. A mechanism that amplifies initial leukocyte accumulation on P-selectin in vitro. J. Clin. Invest., 1996. 98:1081-1087. PMID: 8787668.
76. Bargatze RF, Kurk S, Butcher EC, Jutila MA. Neutrophils roll on adherent neutrophils bound to cytokine-induced endothelial cells via L-selectin on the rolling cells. J Exp Med., 1994. Nov 1;180(5):1785-92. PMID: 7525838.
77. Jung U and Ley K. Mice Lacking Two or All Three Selectins Demonstrate Overlapping and Distinct Functions for Each Selectin. J. Immunol., 1999. 162: 6755-6762. PMID: 10352295.
78. Antibody Engineering, 2nd edition., Borrebaeck CA, Ed., Oxford University Press (1995).
79. Sambrook J and Russell D. Molecular Cloning: A Laboratory Manual, 2nd edition, Cold Spring Harbor Laboratory Press (1989).
80. Bodansky M and Bodansky A. The Practice of Peptide Synthesis, 2nd edition., Springer-Verlag, Berlin. 1995.
81. Altschul SF, Gish W, Miller W, Myers EW, Lipman DJ. Basic local alaignment search tool. J. Mol. Biol., 1990. 215:403-410 PMID: 2231712.
82. Needleman SB, Wunsch CD. A general method applicable to the search for similarities in the amino acid sequence of two proteins. J. Mol. Biol., 1970. 48(3):444-453 PMID: 5420325.
83. Meyers EW and Miller W. Optimal alignments in linear space. Comput. Appl. Biosci., 1988. 4(1):11-17. PMID: 3382986.
84. Tatusova TA and Madden TL. BLAST 2 Sequences, a new tool for comparing protein and nucleotide sequences. FEMS Microbiol. Lett., 1999. 174(2):247-250. PMID: 10339815.
85. Kohler G and Milstein C. Continuous cultures of fused cells secreting antibody of predefined specificity. Nature, 1975. 256(5517): 495-7. PMID: 1172191.
86. Ausubel FM. Current Protocols in Molecular Biology, Greene Publishing Associates and Wiley Interscience, N.Y., 1989).
87. Wigler M, Pellicer A, Silverstein S, Axel R. Biochemical transfer of single-copy eukaryotic genes using total cellular DNA as donor. Cell, 1978. 14(3):725-31. PMID: 210957.
88. Neumann E, Schaefer-Ridder M, Wang Y, Hofschneider PH. EMBO, 1982. J. 1(7):841-5. PMID: 6329708.
89. Harlow E and Lane D. Antibodies: A Laboratory Manual, Cold Spring Harbor Laboratory Press, New York, 1988.
90. Geng JG, Moore KL, Johnson AE, McEver RP. Neutrophil recognition requires a Ca2+-induced conformational change in the lectin domain of GMP-140. J. Biol. Chem., 1991. 266(33): 22313-22318. PMID: 1718992.
91. Mehta P, Patel KD, Laue TM, Erickson HP, Mcever RP. Souluble monomeric P-selectin containing only the lectin and epidermal growth factor domains binds to P-selectin glycoprotein ligand-1 on leukocytes. Blood, 1997. Sep. 15; 90(6): 2381-9. PMID: 9310489.
92. Hirose M, Kawashima H, Miyasaka M. A functional epitope on P-selectin that supports binding of P-selectin to P-selectin glycoprotein ligand-1 but not to sialyl Lewis X oligosaccharides. Int. Immunol., 1998. May; 10(5): 639-49. PMID: 9645612.
93. Ruchaud-Sparangano MH, Malaud E, Gayet O, Chignier E, Buckland R, McGregor JL. Mapping the epitope of a functional P-selectin monoclonal antibody (LYP20) to a short complement-like repeat (SCR 4) domain: use of human-mouse chimera and homologue-replacement mutagenesis. Biochem. J., 1998. 1;332(Pt2):309-14. PMID: 9601057.
94. Chestnut, RW, Polley, MJ, Paulson, JC, Hones, T, Saldanha, JW, Bendig, MM, Kriegler, M. Perez, C, Bayer, R Nunn, M. Antibodies to P-selectin and Their Uses. US Patent No. 5,800,815. Sep. 1, 1998.
95. Berg EL, Fromm C, Melrose J, Tsurushita N. Antibodies cross-reactive with E- and P-selectin block both E- and P-selectin functions. Blood, 1995. Jan 1;85(1):31-7. PMID: 7528571.
96. Leppanen A, Mehta P, Ouyang YB, Ju T, Helin J, Moore KL, van Die I, Canfield WM, McEver RP, Cummings RD. 1999. A novel glycosulfopeptide binds to P-selectin and inhibits leukocyte adhesion to P-selectin. J Biol Chem Aug 27;274(35):24838-48. PMID: 10455156.

## Claims

1. An antibody or binding fragment thereof which specifically binds to a conformational epitope of P-selectin which is within amino acid positions 1-35 of SEQ ID NO:1 and wherein said antibody or binding fragment thereof blocks the binding of PSGL-1 to P-selectin and dissociates preformed P-selectin/PSGL-1 complexes for use for dissociation of preformed P-selectin/PSGL-1 complexes in the treatment of an inflammatory or thrombotic condition or disorder in a subject in need thereof.

2. An antibody or binding fragment thereof for use according to claim 1, wherein the inflammatory or thrombotic condition or disorder is at least one or more selected from the group consisting of: vasoocclusive sickle cell pain crisis, thrombosis, atherosclerosis, tumor metastasis, allergic reactions, thyroiditis, psoriasis, dermatitis, nephritis, lupus erythematosis, scleroderma, sepsis, rhinitis, anaphylaxis, diabetes, multiple sclerosis, graft rejection, graft vs. host disease, asthma, chronic obstructive pulmonary disease, inflammatory bowel disease, arthritis, and ischemic reperfusion injury, conditions associated with extensive trauma, or chronic inflammation, systematic inflammatory response syndrome, and multiple organ failure.

3. An antibody or binding fragment thereof for use according to any preceding claim, wherein the antibody is able to cause dissociation of cell-cell binding between activated endothelial cells and leukocytes, lymphocytes, sickled red cells, and/or platelets.

4. An antibody or binding fragment thereof for use according to any preceding claim, wherein the antibody is able to cause dissociation of cell-cell binding between activated platelets and leukocytes, lymphocytes, sickled red cells, and/or endothelial cells.

5. An antibody or binding fragment thereof for use according to any preceding claim, wherein the antibody is monoclonal.

6. An antibody or binding fragment thereof for use according to any preceding claim, wherein the antibody is chimeric, human, or humanized.

7. An antibody or binding fragment thereof for use according to any preceding claim, wherein the antibody is comprises an immunoglobulin selected from the class consisting of

8. An antibody or binding fragment thereof for use according to any preceding claim, wherein the antibody or binding fragment thereof is an IgG1, IgG2, IgG3, IgG4, or an IgG2/G4 chimera.

9. An antibody binding fragment for use according to any preceding claim, wherein the binding fragment comprises at least one of a Fab, Fab', F(ab)2, or scFv fragment.

10. An antibody binding fragment for use according to any preceding claim, wherein said antibody is for administration to the subject parenterally, intramuscularly, intraperitoneally, epidurally, or orally, intravenously, subcutaneously, or in a nebulized form.

11. An antibody binding fragment for use according to any preceding claim, wherein said antibody or binding fragment is for administration to the subject an amount of 0.1mg/kg to 100 mg/kg, wherein the administration may be oral, via inhalation, subcutaneous, intravenous, intramuscular, intratracheal, intravesical, or intraperitoneal injections and may be given per 24 to 48 hours, per week, every 14 days, or every 4 weeks.

## Patentansprüche

1. Antikörper oder bindendes Fragment davon, der/das sich spezifisch an ein Konformationsepitop von P-Selectin bindet, das innerhalb der Aminosäurepositionen 1 - 35 der SEQ ID Nr:1 ist und wobei der Antikörper oder das bindende Fragment davon die Bindung von PSGL-1 an P-Selectin blockiert und vorgeformte P-Selectin/PSGL-1-Komplexe zur Verwendung zur Dissoziation von vorgeformten P-Selectin/PSGL-1-Komplexen in der Behandlung eines entzündlichen oder thrombotischen Zustands oder Erkrankung in einem Individuum, das diese benötigt, dissoziiert.

2. Antikörper oder bindendes Fragment davon zur Verwendung nach Anspruch 1, wobei der entzündliche oder thrombotische Zustand oder Erkrankung zumindest eines oder mehrere ausgewählt aus der Gruppe bestehend aus: vasookklusiver Sichelzell-Schmerzkrise, Thrombose, Atherosklerose, Tumormetastase, allergischen Reaktionen, Thyreoiditis, Psoriasis, Dermatitis, Nephritis, Lupus erythematodes, Sklerodermie, Sepsis, Rhinitis, Anaphylaxie, Diabetes, Multipler Sklerose, Transplantatabstoßung, Graft-versus-Host-Krankheit, Asthma, chronisch obstruktiver Lungenerkrankung, entzündlicher Darmerkrankung, Arthritis und ischämischer Reperfusionsverletzung, Zustände in Zusammenhang mit augedehnten Traumen oder chronischer Entzündung, systematisches Entzündungsreaktions-Syndrom und multiplem Organversagen ist.

3. Antikörper oder bindendes Fragment davon zur Verwendung nach einem der vorhergehenden Ansprüche, wobei der Antikörper in der Lage ist, die Dissoziation der Zell-Zellbindung zwischen aktivierten Endothelzellen und Leukozyten, Lymphozyten, rote Sichelzellen und/oder Blutplättchen zu bewirken.

4. Antikörper oder bindendes Fragment davon zur Verwendung nach einem der vorhergehenden Ansprüche, wobei der Antikörper in der Lage ist, die Dissoziation der Zell-Zellbindung zwischen aktivierten Blutplättchen und Leukozyten, Lymphozyten, rote Sichelzellen und/oder Endothelzellen zu bewirken.

5. Antikörper oder bindendes Fragment davon zur Verwendung nach einem der vorhergehenden Ansprüche, wobei der Antikörper monoklonal ist.

6. Antikörper oder bindendes Fragment davon zur Verwendung nach einem der vorhergehenden Ansprüche, wobei der Antikörper chimär, human oder humanisiert ist.

7. Antikörper oder bindendes Fragment davon zur Verwendung nach einem der vorhergehenden Ansprüche, wobei der Antikörper ein Immunoglobulin ausgewählt aus der Klasse bestehend aus IgA, IgD, IgE, IgG und IgM ist.

8. Antikörper oder bindendes Fragment davon zur Verwendung nach einem der vorhergehenden Ansprüche, wobei der Antikörper oder das bindende Fragment eine IgG1, IgG2, IgG3, IgG4 oder eine IgG2/G4 Chimäre ist.

9. Antikörper oder bindendes Fragment davon zur Verwendung nach einem der vorhergehenden Ansprüche, wobei das bindende Fragment zumindest eines von einem Fab, Fab', F(ab)2, oder scFv Fragment umfasst.

10. Anitkörper bindendes Fragment zur Verwendung nach einem der vorhergehenden Ansprüche, wobei der Antikörper zur Verabreichnung an das Individuum in parenteraler, intramuskulärer, intraperitonealer, epiduraler oder oraler, intravenöser, subkutaner oder in einer zerstäubten Form vorgesehen ist.

11. Anitkörper bindendes Fragment zur Verwendung nach einem der vorhergehenden Ansprüche, wobei der Antikörper oder das bindende Fragment zur Verabreichnung an das Individuum in einer Menge von 0,1 mg/kg bis 100 mg/kg vorgesehen ist, wobei die Verabreichung oral, durch Inhalation, subkutan, intravenös, intramuskulär, intratracheal, intravesikal oder intraperitoneal Injektionen sein kann und alle 24 bis 48 Stunden, jede Woche, 14-tägig oder alle 4 Woche verabreicht werden kann.

## Revendications

1. Anticorps ou fragment de liaison de celui-ci qui se lie spécifiquement à un épitope conformationnel de P-sélectine qui se trouve dans les positions d'acides aminés 1 à 35 de la SEQ ID n° : 1 et ledit anticorps ou fragment de liaison de celui-ci bloquant la liaison du PSGL-1 à la P-sélectine et dissociant les complexes P-sélectine/PSGL-1 préformés afin d'être utilisé pour la dissociation de complexes P-sélectine/PSGL-1 préformés dans le traitement d'un état ou d'un trouble thrombotique ou inflammatoire chez un sujet en ayant besoin.

2. Anticorps ou fragment de liaison de celui-ci selon la revendication 1, ledit état ou trouble thrombotique ou inflammatoire étant au moins un ou plusieurs choisis dans le groupe constitué par : une crise de douleur vaso-occlusive à cellules falciformes, une thrombose, une athérosclérose, des métastases tumorales, des réactions allergiques, la thyroïdite, le psoriasis, une dermatite, la néphrite, le lupus érythémateux, la sclérodermie, une septicémie, une rhinite, l'anaphylaxie, le diabète, la sclérose en plaques, un rejet de greffe, des maladies causées par la réaction du greffon contre l'hôte, l'asthme, la bronchopneumopathie chronique obstructive, une maladie intestinale inflammatoire, l'arthrite, et des lésions de reperfusion ischémique, des états associés à un traumatisme extensif, ou une inflammation chronique, un syndrome de réponse inflammatoire systématique, et le syndrome de défaillance multiviscérale.

3. Anticorps ou fragment de liaison de celui-ci destiné à être utilisé selon l'une quelconque des revendications précédentes, ledit anticorps pouvant entraîner la dissociation des liaisons cellule-cellule entre des cellules endothéliales activées et des leucocytes, des lymphocytes, des globules rouges falciformes et/ou des plaquettes.

4. Anticorps ou fragment de liaison de celui-ci destiné à être utilisé selon l'une quelconque des revendications précédentes, ledit anticorps pouvant entraîner la dissociation des liaisons cellule-cellule entre des plaquettes activées et des leucocytes, des lymphocytes, des globules rouges falciformes et/ou des cellules endothéliales.

5. Anticorps ou fragment de liaison de celui-ci destiné à être utilisé selon l'une quelconque des revendications précédentes, ledit anticorps étant monoclonal.

6. Anticorps ou fragment de liaison de celui-ci destiné à être utilisé selon l'une quelconque des revendications précédentes, ledit anticorps étant chimérique, humain ou humanisé.

7. Anticorps ou fragment de liaison de celui-ci destiné à être utilisé selon l'une quelconque des revendications précédentes, ledit anticorps comprenant une immunoglobuline choisie dans la catégorie constituée par l'IgA, l'IgD, l'IgE, l'IgG et l'IgM.

8. Anticorps ou fragment de liaison de celui-ci destiné à être utilisé selon l'une quelconque des revendications précédentes, ledit anticorps ou fragment de liaison de celui-ci étant une IgG1, une IgG2, une IgG3, une IgG4 ou une chimère IgG2/G4.

9. Anticorps ou fragment de liaison de celui-ci destiné à être utilisé selon l'une quelconque des revendications précédentes, ledit fragment de liaison comprenant au moins un fragment parmi un fragment Fab, Fab', F(ab)2 ou scFv.

10. Fragment de liaison d'anticorps destiné à être utilisé selon l'une quelconque des revendications précédentes, ledit anticorps étant destiné à une administration à un sujet par voie parentérale, intramusculaire, intrapéritonéale, épidurale, ou par voie orale, intraveineuse, sous-cutanée ou sous forme nébulisée.

11. Fragment de liaison d'anticorps destiné à être utilisé selon l'une quelconque des revendications précédentes, ledit anticorps ou fragment de liaison étant destiné à l'administration à un sujet d'une quantité de 0,1 mg/kg à 100 mg/kg, ladite administration pouvant être orale, par inhalation, sous-cutanée, intraveineuse, intramusculaire, intratrachéale, intra-vésicale ou des injections intrapéritonéales et pouvant être donnée toutes les 24 à 48 heures, une fois par semaine, tous les 14 jours ou toutes les 4 semaines.
